# EUROPÄISCHE PATENTSCHRIFT

(11) **EP 0 848 703 B1**
(45) Veröffentlichungstag und Bekanntmachung des Hinweises auf die Patenterteilung: **27.09.2000**
(21) Anmeldenummer: 96922910.3
(22) Anmeldetag: 05.07.1996
(51) Int. Cl.: C07C 311/39, C07C 323/67, C07C 311/48, C07C 311/40, C07D 275/06, C07D 333/34, C07D 213/76, A61K 31/18

(54) **DERIVATE VON BENZOSULFONAMIDEN ALS HEMMER DES ENZYM CYCLOOXYGENASE II**
DERIVATIVES OF BENZOSULPHONAMIDES AS INHIBITORS OF THE ENZYME CYCLO-OXYGENASE II
DERIVES DE BENZOSULFONAMIDES UTILISES COMME INHIBITEURS DE LA CYCLOOXYGENASE II

(30) Priorität: 21.07.1995 AT 124295; 21.07.1995 AT 124395
(43) Veröffentlichungstag der Anmeldung: 24.06.1998
(73) Patentinhaber: Nycomed Austria GmbH, 4021 Linz (AT)
(72) Erfinder: BLASCHKE, Heinz, A-4020 Linz (AT); KREMMINGER, Peter, A-4481 Asten (AT); HARTMANN, Michael, A-4040 Linz (AT); FELLIER, Harald, A-4040 Linz (AT); BERG, Jörg, A-4040 Linz (AT); CHRISTOPH, Thomas, D-52080 Aachen (DE); ROVENSZKY, Franz, A-4020 Linz (AT); STIMMEDER, Dagmar, A-4020 Linz (AT)
(74) Vertreter: Matthews, Derek Peter
(86) Internationale Anmeldenummer: EP9602954
(87) Internationale Veröffentlichungsnummer: WO9703953

(56) Entgegenhaltungen:
- WO-A-94/13635
- US-A- 3 906 024

## Beschreibung

Die Erfindung betrifft neue Derivate von Benzolsulfonsäuren mit antiinflammatorischer Wirkung.

Prostaglandine spielen eine entscheidende Rolle in Entzündungsprozessen und die Hemmung der Prostaglandinbildung, speziell der Bildung von PGG₂, PGH₂ und PGE₂ ist die gemeinsame Eigenschaft von antiinflammatorisch wirkenden Verbindungen. Die bekannten nicht steriodalen antiinflammatorischen Wirkstoffe (NSAIDs), die bei Entzündungsprozessen Prostaglandininduzierten Schmerz und Schwellungen reduzieren, beeinflussen auch Prostaglandin-regulierte Prozesse, die nicht mit Entzündungsprozessen einhergehen. Daher verursachen die meisten bekannten NSAIDs in höheren Dosen unerwünschte Nebenwirkungen, oft sogar lebensbedrohende Geschwüre, insbesondere Magengeschwüre, Magenblutungen und dergleichen. Dadurch wird das therapeutische Potential dieser Verbindungen entscheidend eingeschränkt.

Die meisten bekannten NSAIDs hemmen die Bildung von Prostaglandinen durch Hemmung von Enzymen im menschlichen Arachidonsäure-Stoffwechsel, insbesondere durch Hemmung des Enzyms Cyclooxygenase (COX). Ein erst vor kurzem entdecktes Enzym des menschlichen Arachidonsäure-Stoffwechsels ist das Enzym Cyclooxygenase II (COX-2). (Proc. Natl. Acad. Sci. USA, 89, 7384, 1992). COX-2 wird durch Cytokine oder Endotoxine induziert. Die Entdeckung dieses induzierbaren Enzyms, das in Entzündungsprozessen eine entscheidende Rolle spielt, eröffnet die Möglichkeit selektiv wirksame Verbindungen mit antiinflammatorischer Wirkung zu suchen, die den Entzündungsprozeß wirksamer hemmen ohne andere Prostaglandin-regulierte Prozesse zu beeinflussen und dabei weniger und weniger schwere Nebenwirkungen aufweisen.

Aus WO 94/13635 sind 5-Methylsulfonamid-1-indanone bekannt, die das Enzym Cyclooxygenase II hemmen und daher bei der Behandlung von Entzündungsprozessen eingesetzt werden können. Das Potential und die Nebenwirkungen dieser Verbindungen sind bisher noch nicht zur Gänze abgeklärt. Ferner sind diese bekannten Verbindungen schlecht löslich und weisen daher entscheidende Nachteile bei der Formulierung und Anwendung auf. Es besteht daher nach wie vor der Bedarf an neuen Cyclooxygenase II-selektiven Verbindungen, die durch ihr Wirk- und Nebenwirkungsprofil sicher und effizient in der Anwendung bei der Behandlung von entzündlichen Prozessen sind.

Aufgabe der vorliegenden Erfindung war daher die Bereitstellung neuer nichtsteroidaler Entzündungshemmer (NSAIDs), die selektiv Cyclooxygenase II (COX-2) hemmen, und daher weniger und weniger schwere unerwünschte Nebenwirkungen aufweisen.

Diese Aufgabe konnte unerwarteterweise durch die Bereitstellung von neuen Derivaten von Benzolsulfonsäuren gelöst werden. Diese neuen Verbindungen weisen durch ihre selektive Wirkung auf das Enzym Cyclooxygenase II ausgezeichnete antiinflammatorische analgetische, antipyretrische und antiallergische Wirkung auf ohne jedoch die äußerst unerwünschten Nebenwirkungen bekannter Antiinflammatorika aufzuweisen.

Gegenstand der Erfindung sind daher Verbindungen der Formel I 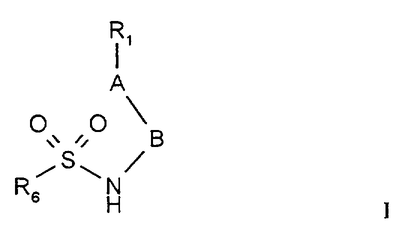 in der
- A: Sauerstoff, Schwefel oder NH.
- R₁: einen gegebenenfalls ein- oder mehrfach durch Halogen, Alkyl, CF₃ oder Alkoxy substituierter Cycloalkyl-, Aryl- oder Heteroarylrest

B eine Gruppe der Formel II a oder IIb 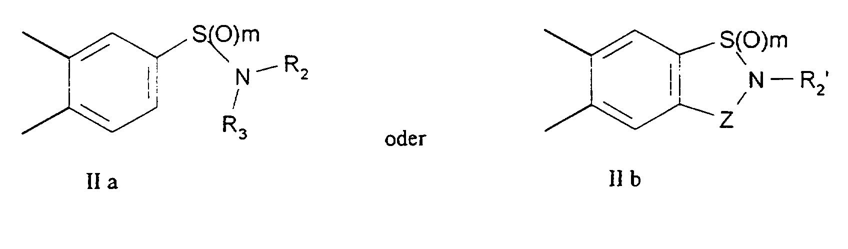
R₂ und R₃ unabhängig voneinander Wasserstoff, einen gegebenenfalls polyfluorierten Alkylrest, einen Aralkyl- Aryl- oder Heteroarylrest oder einen Rest (CH₂)ₙ-X.
   oder
R₂ und R₃ gemeinsam mit dem N- Atom einen drei bis siebengliedrigen, gesättigten, teilweise oder vollständig ungesättigten Heterocyclus mit einem oder mehreren Heteroatomen N, O, oder S, der gegebenenfalls durch Oxo, einen Alkyl-, Alkylaryl- oder Arylrest oder einen Rest (CH₂)ₙ-X substituiert sein kann,
R₂' Wasserstoff, einen gegebenenfalls polyfluorierten Alkylrest, einen Aralkyl- Aryl- oder Heteroarylrest oder einen Rest (CH₂)ₙ-X,
   wobei
X Halogen, NO₂, -OR₄, -COR₄, -CO₂R₄, -OCO₂R₄, -CN, -CONR₄OR₅, -CONR₄R₅,-SR₄, -S(O)R₄, -S(O)₂R₄, -NR₄R₅, -NHC(O)R₄, -NHS(O)₂R₄
Z -CH₂-, -CH₂-CH₂-, -CH₂-CH₂-CH₂-, -CH₂-CH=CH-, -CH=CH-CH₂-, -CH₂-CO-, -CO-CH₂₋, -NHCO-, - -CONH-, -NHCH₂₋, -CH₂NH-, -N=CH-, -NHCH-, -CH₂-CH₂-NH-, -CH=CH-, >N-R₃, >C=O, >S(O)ₘ.
R₄ und R₅ unabhängig voneinander Wasserstoff, Alkyl, Aralkyl oder Aryl,
n eine ganze Zahl von 0 bis 6,
R₆ einen geradkettigen oder verzweigten Alkylrest mit 1 - 4 C- Atomen, der gegebenenfalls durch Halogen oder Alkoxy ein- oder mehrfach substituiert sein kann, oder CF₃ und
m eine ganze Zahl von 0 bis 2
bedeuten,
mit der Bedingung, daß A nicht O bedeutet, wenn R₆CF₃ bedeutet,
und deren pharmazeutisch verträgliche Salze.

A bedeutet Sauerstoff, Schwefel oder NH.

R₁ bedeutet einen Cycloalkylrest, beispielsweise einen Cyclohexyl- oder einen Cyclopentylrest, einen Arylrest, beispielsweise einen Phenylrest, oder Heteroarylrest, beispielsweise einen Furyl, Thienyl-, Thiazolyl-, Imidazolyl-, Thiadiazolyl-, Pyridyl-, Pyrazolylrest.
Diese Reste können gegebenenfalls ein oder mehrfach durch Halogen, beispielsweise Cl, F, Br, oder durch CF₃ oder Alkyl mit 1 - 4 C-Atomen, beispielsweise Methyl, Ethyl, Propyl, iso- Propyl, Butyl, iso-Butyl oder tertiär-Butyl oder Alkoxy mit 1 - 4 C- Atomen, beispielsweise Methoxy. Ethoxy, Propoxy oder Butoxy substituiert sein.

R₂ und R₃ bedeuten unabhängig voneinander Wasserstoff, einen gegebenenfalls polyfluorierten Alkylrest mit 1 - 6 C-Atomen, beispielsweise Methyl-, einen Ethyl-, einen Propyl-, einen iso-Propyl, einen Butyl,- einen iso-Butyl-, einen tertiär-Butyl-. einen Pentyl-, einen iso-Pentyl-, einen Hexyl- oder einen iso-Hexylrest, einen Rest CF₃ oder C₂F₅, einen Aralkylrest mit 1- 4 C-Atomen in der Alkylkette, beispielsweise einen Benzylrest, einen Ethylphenylrest, einen Arylrest, beispielsweise einen Phenylrest oder einen Heteroarylrest, beipielsweise einen Pyridylrest, einen Pyridazinylrest, einen Thienylrest, einen Thiazoylrest oder einen Isothiazolylrest.

R₂ und R₃ können ferner unabhängig voneinander einen Rest -(CH₂)ₙ-X, bedeuten, wobei X Halogen, -NO₂, -OR₄, -COR₄, -CO₂R₄, - OCO₂R₄, -CN, -CONR₄OR₅, -CONR₄R₅, -SR₄,-S(O)R₄, -S(O)₂R₄, -NR₄R₅, -NHC(O)R₄, -NHS(O)₂R₄, und n eine ganze Zahl von 0 bis 6 ist.
Beispiele für solche Reste sind Halogenalkylreste, beispielsweise Chlormethyl, Chlorethyl, der Rest -CN, Nitroalkylreste, beispielsweise Nitromethyl-, Nitro-ethyl, oder Cyanoalkylreste, beispielsweise Cyanomethyl, Cyanopropyl, Cyanohexyl, ein Hydroxyrest oder Hydroxyalkylreste, beispielsweise Hydroxmethyl, Hydroxethyl, Hydroxypropyl Bishydroxymethyl-methyl, Weitere Beispiele sind Alkoxyreste wie Methoxy, Ethoxy, Propoxy, Butoxy, Pentoxy, die Reste Methyloxy-ethyl, Ethyloxy-methyl, Carbonsäurereste wie Ethoxycarbonyl,- Methoxycarbonyl, Acetyl-, Propionyl-, Butyryl-, Isobutyrylreste und deren Alkyl-. Aralkyl- oder Aryl-ester, Carbamoylreste, Oxycarbonyloxyreste, beispielsweise der Ethoxycarbonyloxyrest, Carboximidsäurereste, Thiocarboxyreste und dergleichen.

Z-bedeutet -CH₂-, -CH₂-CH₂-, -CH₂-CH₂-CH₂-, -CH₂-CH=CH-, -CH=CH-CH₂-, -CH₂-CO-,-CO-CH₂₋, -NHCO-, - -CONH-, -NHCH₂₋, -CH₂NH-, -N=CH-, -NHCH-, -CH₂-CH₂-NH-,-CH=CH-, >N-R₃, >C=O, >S(O)ₘ, wobei m eine ganze Zahl von 0 bis 2 bedeutet

R₂' bedeutet Wasserstoff, einen gegebenenfalls polyfluorierten Alkylrest mit 1 - 6 C-Atomen, beispielsweise Methyl-, einen Ethyl-, einen Propyl-. einen iso- Propyl, einen Butyl,- einen iso-Butyl-, einen tertiär-Butyl-, einen Pentyl-, einen iso-Pentyl-, einen Hexyl- oder einen iso-Hexylrest, einen Rest CF₃ oder C₂F₅ einen Aralkylrest mit 1-4C- Atomen in der Alkylkette, beispielsweise einen Benzylrest, einen Ethylphenylrest, einen Arylrest, beispielsweise einen Phenylrest oder einen Heteroarylrest, beipielsweise einen Pyridylrest, einen Pyridazinylrest, einen Thienylrest, einen Thiazoylrest oder einen Isothiazolylrest.
R₂' kann ferner einen Rest -(CH₂)ₙ-X, bedeuten, wobei X Halogen, -NO₂, -OR₄, -COR₄,-CO₂R₄, - OCO₂R₄ -CN, -CONR₄OR₅, -CONR₄R₅, -SR₄, -S(O)R₄, -S(O)₂R₄, -NR₄R₅,-NHC(O)R₄, -NHS(O)₂R₄, und n eine ganze Zahl von 0 bis 6 ist

R₄ und R₅ bedeuten unabhängig voneinander Wasserstoff, Alkyl mit 1 - 6 C-Atomen. Aralkyl mit 1 - 4 C-Atomen in der Alkylkette, beispielsweise Benzyl, Ethylphenyl oder Aryl, beispielsweise Phenyl.

Weiters können R₂ und R₃ gemeinsam mit dem N- Atom einen drei- bis siebengliedrigen, gesättigten, teilweise oder vollständig ungesättigten Heterocyclus mit einem oder mehreren Heteroatomen N, O, oder S, bilden, der gegebenenfalls durch Oxo, einen Alkyl-, Alkylaryl- oder Arylrest oder einen Rest (CH₂)ₙ-X wobei X Halogen, NO₂, -OR₄, -COR₄, -CO₂R₄, - OCO₂R₄, -CN, -CONR₄OR₅, -CONR₄R₅, -SR₄, -S(O)R₄, -S(O)₂R₄, -NR₄R₅, -NHC(O)R₄, -NHS(O)₂R₄-, und n eine ganze Zahl von 0 bis 6 bedeutet, substituiert sein kann.
Beispiele für solche Ringe sind der Morpholylrest, der Aziridinylrest, der Azetidinylrest, der Pyridylrest, der Pyrazolylrest, der Thiazolylrest und dergleichen.

R₆ bedeutet einen geradkettigen oder verzweigten Alkylrest mit 1 - 4 C-Atomen, beispielsweise Methyl, Ethyl, Propyl, iso-Propyl, Butyl, iso-Butyl oder tertiär-Butyl. Diese Reste können gegebenenfalls ein oder mehrfach durch Halogen, beispielsweise Cl, F, oder Br, oder Alkoxy, beispielsweise Methoxy, Ethoxy und dergleichen substituiert sein.

Die erfindungsgemäßen Verbindungen, in denen B eine Gruppe der Formel II a bedeutet kön,nen hergestellt werden, indem man eine Verbindung der Formel III 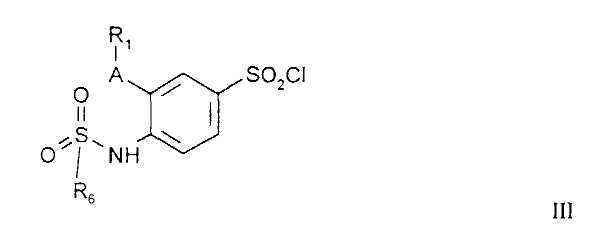 mit einer Verbindung der Formel IV

HNR₂R₃ (IV)

oder deren Salz umsetzt.

Die Umsetzung erfolgt vorzugsweise in Gegenwart eines unter Reaktionsbedingungen inerten Verdünnungs- oder Lösungsmittels, beispielsweise Dioxan, Tetrahydrofuran oder dergleichen, Die Reaktionstemperatur beträgt etwa -10°C bis zur Rückflußtemperatur des Lösungs- oder Verdünnungsmittels, vorzugsweise -10° C bis Raumtemperatur.

Die Ausgangsverbindungen der Formel III können beispielsweise nach folgendem Reaktionsschema oder durch andere dem Fachmann geläufige Methoden hergestellt werden.

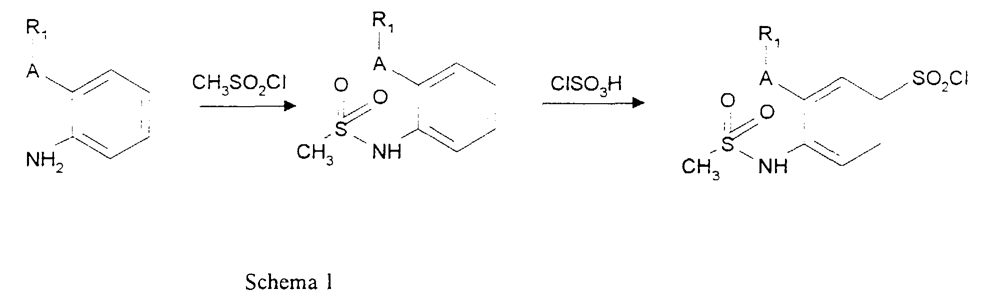

Weiters können die Verbindungen der Formel I, in denen B eine Gruppe IIa bedeutet nach folgendem Reaktionsschem hergestellt werden:

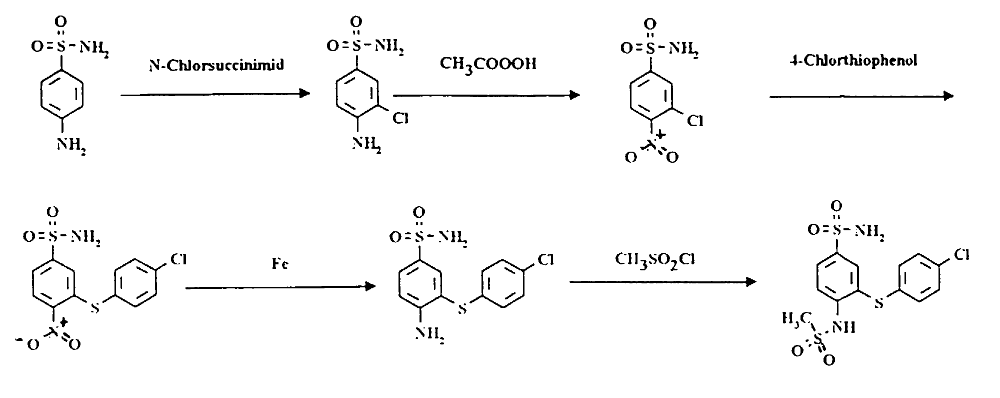

Die erfindungsgemäßen Verbindungen, in denen B eine Gruppe der Formel II b können hergestellt werden indem man eine Verbindung der Formel V 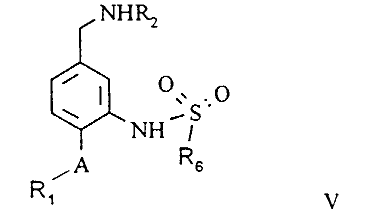 mit einem Säurehalogenid des Schwefels, beispielsweise Sulfurylchlorid umsetzt.

Die Umsetzung erfolgt vorzugsweise in Gegenwart eines unter Reaktionsbedingungen inerten Verdünnungs- oder Lösungsmittels, beispielsweise Dioxan, Tetrahydrofuran oder dergleichen, vorzugsweise in Gegenwart eines Katalysators, beispielsweise Aluminiumchlorid. Die Reaktionstemperatur beträgt etwa -10°C bis zur Rückflußtemperatur des Lösungs- oder Verdünnungsmittels, vorzugsweise -10° C bis Raumtemperatur.

Die Ausgangsverbindungen der Formel V können beispielsweise nach folgendem Reaktionsschema oder durch andere dem Fachmann geläufige Methoden hergestellt werden.

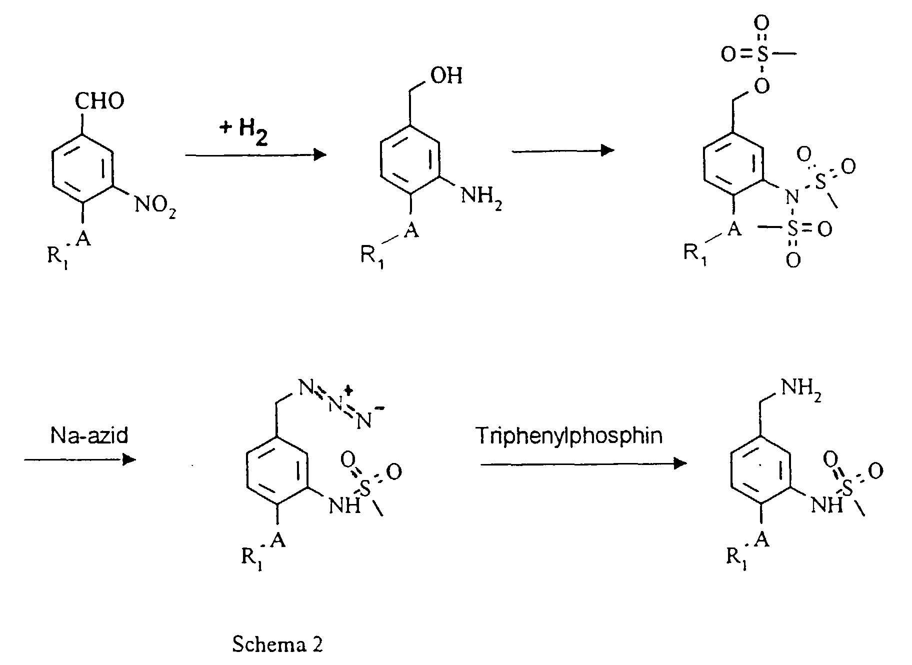

Die erfindungsgemäßen Verbindungen der Formel I, in denen B eine Gruppe der Formel IIb bedeutet können alternativ auf folgendem Weg hergestellt werden:

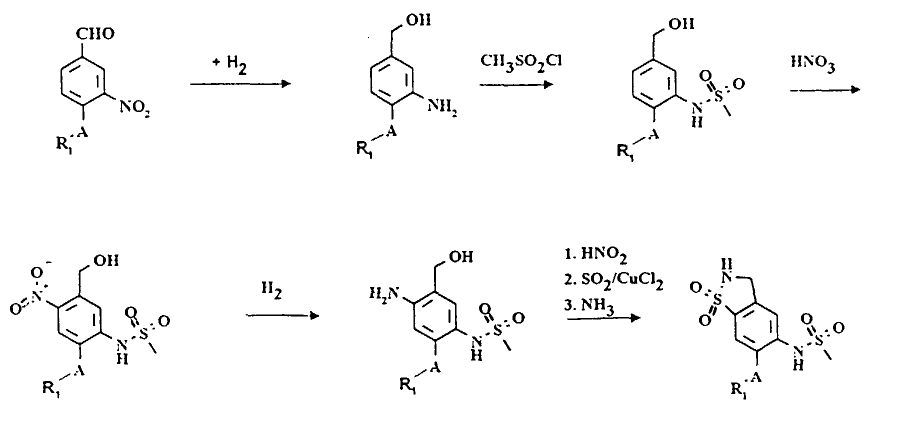

Die auf die oben beschriebene Weise erhaltenen Verbindungen der Formel I sind saure oder basische Verbindungen und können auf übliche Weise mit anorganischen oder organischen Basen bzw. Säuren in ihre pharmazeutisch verträglichen Salze überführt werden. Die Salzbildung kann beispielsweise durchgeführt werden, indem man eine Verbindung der Formel I in einem geeigneten Lösungsmittel, wie beispielsweise Wasser, Aceton, Acetonitril, Benzol, Dimethylformamid, Dimethylsulfoxid, Chloroform, Dioxan, Methanol, Ethanol, Hexanol, Ethylacetat oder in einem aliphatischen Ether, beispielsweise Diethylether, oder Mischungen solcher Lösungsmittel löst. eine zumindest äquivalente Menge der gewünschten Base oder Säure zusetzt, für eine gute Durchmischung sorgt und nach beendeter Salzbildung das ausgefallene Salz abfiltriert, lyophilisiert oder das Lösungsmittel im Vakuum abdestilliert. Gegebenenfalls können die Salze nach der Isolierung umkristallisiert werden.

Pharmazeutisch verträgliche Salze sind solche mit anorganischen Säuren, wie beispielsweise Salzsäure, Bromwasserstoffsäure. Schwefelsäure, Phosphorsäure oder Salpetersäure, oder mit organischen Säuren wie Zitronensäure, Weinsäure, Maleinsäure, Fumarsäure, Bemsteinsäure, Äpfelsäure, Methansulfonsäure, Aminosulfonsäure, Essigsäure. Benzoësäure und dgl.
Pharmazeutisch verträgliche Salze sind z.B. Metallsalze, insbesondere Alkalimetall- oder Erdalkalimetallsalze, wie Narium-, Kalium-, Magnesium- oder Calciumsalze. Andere pharmazeutische Salze sind beispielsweise leicht kristallisierende Ammoniumsalze. Diese leiten sich von Ammoniak oder organischen Aminen, wie Mono-, Di- oder Tri-nieder-(alkyl, cykloalkyl oder Hydroxyalkyl)-aminen, Niederalkylendiaminen oder Hydroxy- oder Arylniederalkylammoniumbasen, z.B. Methylamin, Diethylamin, Triethylamin, Ethylendiamin, Tris-(hydroxymethyl)-aminomethan, Benzyltrimethylammonium-hydroxid und dergleichen ab.

Die neuen Verbindungen sind gut löslich und weisen durch ihre selektive Wirkung auf das Enzym Cyclooxygenase II ausgezeichnete antiinflammatorische. analgetische, antipyretrische und antiallergische Wirkung auf, ohne jedoch die äußerst unerwünschten Nebenwirkungen bekannter Antiinflammatorika aufzuweisen.

Aufgrund dieser pharmakologischen Eigenschaft können die neuen Verbindungen allein oder in Verbindung mit anderen Wirksubstanzen in Form üblicher galenischer Zubereitung als Heilmittel zur Behandlung von Störungen oder Krankheiten, die durch Inhibierung der Cyclooxygenase II verhindert, behandelt oder geheilt werden können, eingesetzt werden.

Diese Störungen oder Krankheiten umfassen Schmerz. Fieber und Entzündungen verschiedenster Art. beispielsweise. rheumatisches Fieber- Symptome, die mit Grippe, grippalen oder anderen viralen Infekten einhergehen. Kopf- und Gliederschmerzen. Zahnschmerzen. Verstauchungen. Distorsionen, Neuralgien. Muskelentzündungen. Gelenksentzündungen, Gelenkshautentzündungen, Arthritis, rheumatoide Arthritis, sonstige rheumatische Entzündungsformen degenerative Erscheinungen. beispielsweise Ostheoarthritis, Gicht, Gelenksversteifüng, Spondylitis, Schleimbeutelentzündungen. Verbrennungen und Verletzungen

Die Erfindung bezieht sich daher auch auf pharmazeutische Präparate, welche die erfindungsgemäßen Verbindungen der Formel I oder ihre Salze allein oder gemischt mit anderen therapeutisch wertvollen Wirkstoffen. sowie üblichen galenischen Hilfs- und/oder Trägerstoffen oder Verdünnungsmitteln enthalten.

Die erfindungsgemäßen Verbindungen können in Form von Tabletten oder Kapseln, welche eine Einheitsdosierung der Verbindung zusammen mit Hilfsstoffen und Verdünnungsmitteln wie Maisstärke, Calciumcarbonat, Dicalciumphosphat. Alginsäure, Lactose. Magnesiumstearat, Primogel oder Talkum enthalten, oral appliziert werden. Die Tabletten werden in herkömmlicher Weise durch Granulieren der Inhaltsstoffe und Pressen, die Kapseln durch Einfüllen in Hartgelatinekapseln geeigneter Größe hergestellt.
Eine weitere Applikationsform der erfindungsgemäßen Verbindungen sind Suppositorien, die Hilfsstoffe, wie Bienenwachsderivate, Polyethylenglykol oder Polyethylenglykolderivate, Linol- oder Linolensäureester, zusammen mit einer Einheitsdosierung der Verbindung enthalten und rektal verabreicht werden können.

Die erfindungsgemäßen Verbindungen können auch parenteral, beispielsweise durch intramuskuläre, intravenöse oder subkutane Injektion, appliziert werden. Für die parenterale Applikation werden sie am besten in Form einer sterilen wäßrigen Lösung verwendet, welche andere gelöste Stoffe, wie tonische Mittel. Mittel zur Einstellung des pH-Wertes, Konservierungsmittel und Stabilisatoren enthalten können. Die Verbindungen können destilliertem Wasser zugesetzt werden und der pH-Wert kann unter Verwendung von beispielsweise Citronensäure, Milchsäure oder Salzsäure auf 3 bis 6 eingestellt werden. Ausreichend gelöste Stoffe, wie Dextrose oder Salzlösung, können zugesetzt werden, um die Lösung isotonisch einzustellen. Außerdem können Konservierungsmittel, wie p-Hydroxybenzoate, und Stabilisatoren, wie EDTA, zugesetzt werden um eine ausreichende Haltbarkeit und Stabilität der Lösung sicherzustellen. Die so erhaltene Lösung kann dann sterilisiert werden und in sterile Ampullen geeigneter Große, sodaß sie das gewünschte Lösungsvolumen enthalten, gefüllt werden. Die erfindungsgemäßen Verbindungen können auch durch Infusion einer wie oben beschriebenen parenteralen Formulierung appliziert werden.

Femer können die erfindungsgemäßen Verbindungen für die topische oder transdermale Applikation mit geeigneten Hilfs- und/oder Trägerstoffen, Emulgatoren, Tensiden und /oder Verdünnungsmitteln, z.B. Vaseline, Olivenöl. Erdnußöl, Sesamöl, Sojaöl, Wasser, Glykole, Cetylstearylester, Triglyceride, Cetaceum, Miglyol u. dgl. zu Salben, Cremes, Gelen oder Pflastem oder beispielsweise mit Talkum zu Puder formuliert werden.

Für die orale Applikation beim Menschen wird angenommen, daß der tägliche Dosierungswert einer erfindungsgemäßen Verbindung im Bereich von 0.01 bis 1000 mg pro Tag für einen typischen erwachsenen Patienten von 70 kg liegt. Daher können Tabletten oder Kapseln üblicherweise 0,003 bis 300 mg an aktiver Verbindung, beispielsweise 0,1 bis 50 mg, für die orale Applikation bis zu dreimal am Tag enthalten. Bei parenteraler Verabreichung kann die Dosis im Bereich von 0.01 bis 1000 mg pro 70 kg und Tag, zum Beispiel etwa 5 mg, liegen.

### Beispiel 1:

### 3-(2,4-Dichlorphenoxy)-4-methylsulfonylaminobenzolsulfonsäureamid

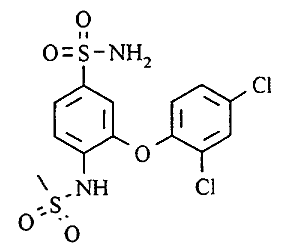

(a) 2-(2,4-Dichlorphenoxy)-nitrobenzol 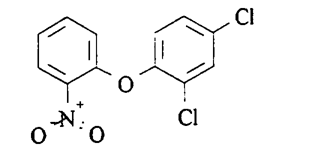 2-Chlomitrobenzol (20.0 g, 126.94 mmol) wurde in Xylol (400 ml) gelöst und 2,4-Dichlorphenol (22.7 g, 139.0 mmol) wurde zugegeben. Anschließend wurde Kaliumcarbonat (19.2 g, 139.0 mmol) zugegeben und die resultierende Mischung 10 h unter Rückfluß erhitzt. Nach weiterer Zugabe von 2,4-Dichlorphenol (6.8 g, 41.7 mmol) und Kaliumcarbonat (5.8 g, 42.0 mmol) wurde noch über Nacht unter Rückfluß erhitzt. Nach dem Abkühlen wurde der feste Rückstand abfiltriert und das Lösungsmittel abgedampft. Der Rückstand wurde aus Ethanol umkristallisiert.
   Ausbeute: 26.2 g = 72.7 %
   ¹³C (100MHz. CDCl₃) δ 150.01, 149.81. 140.81, 134.28, 130.86. 130.77, 128.38, 126.99, 126.02, 123.73, 121.79, 119.22
(b) 2-(2,4-Dichlorphenoxy)-anilin 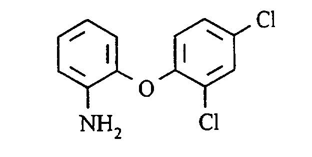 2-(2,4-Dichlorphenoxy)-nitrobenzol (10.0 g, 35.2 mmol) wurde in Dioxan (100 ml) gelöst und eine Suspension von Raney Nickel in Wasser (20 g) wurde zugegeben. Die Mischung wurde 6 h bei 3.5 bis 4.0 bar hydriert. Danach wurde filtriert und vom Lösungsmittel befreit. Ausbeute: 8.9 g = 100 %
   ¹³C (100MHz, CDCl₃) δ 151.79, 142.68, 138.29, 130.31, 128.24, 127.91, 125.45, 125.04, 119.46, 118.89, 118.78, 116.68
(c) 2-(2,4-Dichlorphenoxy)-N-methylsulfonylanilid 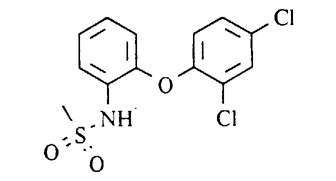 2-(2,4-Dichlorphenoxy)-anilin (8.9 g, 35.02 mmol) wurde in Dichlormethan (200 ml) gelöst und Triethylamin (14.7 g, 145 mmol) wurde bei 0°C zugegeben. Bei dieser Temperatur wurde Methansulfonsäurechlorid (4.0 g, 35.02 mmol) zugetropft. Nach 1 h bei 0°C wurde noch eimal Methansulfonsäurechlorid (4.0 g, 35.02 mmol) zugetropft und eine weitere h gerührt. Die Mischung wurde auf gesättigte NaHCO₃ Lösung gegossen und die Phasen wurden getrennt. Die wässrige Phase wurde noch 2 x mit Dichlormethan extrahiert und die vereinigten organischen Phasen über MgS04 getrocknet. Nach dem Abdampfen des Lösungsmittels wurde der Rückstand in Dioxan (100 ml) und Methanol (100 ml) gelöst und auf 0°C gekühlt. 2N Natronlauge in Wasser (100 ml, 200 mmol) wurde zugetropft und die Lösung wurde 30 min, bei 0°gerührt. Die Mischung wurde mit KHSO₄ angesäuert und mit Ethylacetat extrahiert. Es wurde über MgSO₄ getrocknet und das Lösungsmittel abgedampft. Der Rückstand wurde aus Ethanol umkristallisiert.
   Ausbeute: 9.28 g = 80 %
   ¹³C (100MHz, CDCl₃) δ 149.68, 147.24, 130.88, 130.78, 128.51, 127.30, 126.98, 125.86, 124.44, 122.40, 122.19, 116.09, 39.73
(d) 3-(2,4-Dichlorphenoxy)-4-methylsulfonylaminobenzolsulfonsäurechlorid 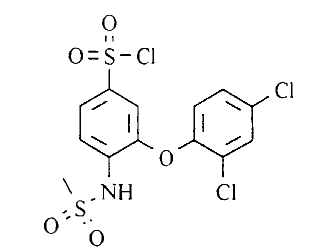 Chlorsulfonsäure (0.80 ml, 12.0 mmol) wurde in Chloroform (10 ml) gelöst und auf 0°C gekühlt. Eine Lösung von 2-(2,4-Dichlorphenoxy)-N-methylsulfonylanilide (1.0 g, 3.01 mmol) in Chloroform (5 ml) wurde zugetropft und die Lösung 30 min. bei 0°C und 2 h bei Raumtemperatur gerührt. Nach der Zugabe von Wasser wurde mit Chloroform extrahiert, über MgSO₄ getrocknet und abrotiert. Der Rückstand wurde chromatographisch gereinigt (Dichlormethan / Petrolether - Kieselgel).
   Ausbeute: 0.50 g = 38.8 %
   ¹³C (100MHz, CDCl₃) δ 147.79, 145.65, 139.20, 133.80, 132.84, 131.51, 129.21, 127.39, 123.43, 118.26, 112.98, 40.85
(e) 3-(2,4-Dichlorphenoxy)-4-methylsulfonylaminobenzolsulfonsäureamid
   Eine Mischung von Dioxan (20 ml) und konz. Salmiak (20 ml) wurde auf 0°C gekühlt und eine Lösung von 3-(2,4-Dichlorphenoxy)-4-methylsulfonyl-aminobenzolsulfonsäurechlorid (0.49 g, 1.14 mmol) in Dioxan (10 ml) wurde zugetropft. Die Lösung wurde 1 h bei 0°C gerührt und anschließend mit konz. HCl angesäuert. Es wurde mit Ethylacetat extrahiert, über MgSO₄ getrocknet und abrotiert. Der Rückstand wurde aus Chloroform umkristallisiert. Ausbeute: 0.37 g = 80%
   ¹³C (100MHz, DMSO-d₆) δ 149.96, 147.96, 141.03, 131.42, 130.40, 129.60, 129.21, 126.33, 123.97, 123.03, 121.57, 114,05, 40.99

### Beispiel 2:

### 6-(2,4-Dichlorphenoxy)-5-methylsulfonylamino-2H-1.2-benzothiazolidin-1,1-dioxid

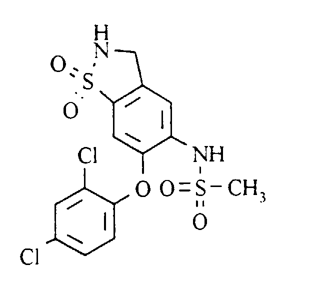

(a) 4-(2,4-Dichlorphenoxy)-3-nitrobenzaldehyd 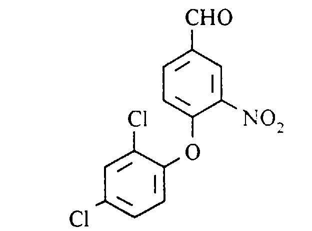 4-Chlor-3-nitrobenzaldehyd (47.12 g, 253.92 mmol) wurde in Xylol (400 ml) gelöst und 2,4-Dichlorphenol (45.32 g, 278.03 mmol) wurde zugegeben. Anschließend wurde Kaliumcarbonat (38.40 g, 277.84 mmol) zugesetzt und die resultierende Mischung 6 h unter Rückfluß erhitzt. Nach dem Abkühlen wurde der feste Rückstand abfiltriert und das Lösungsmittel abgedampft. Der Rückstand wurde in CH₂Cl₂ gelöst, mehrmals mit 1N NaOH extrahiert und über MgSO₄ getrocknet. Nach dem Abdampfen des Lösungsmittels wurde im Vakuum getrocknet. Das Produkt konnte ohne weitere Reinigung für die nächste Stufe eingesetzt werden.
   Ausbeute: 74.5 g = 94%
   ¹³C (100MHz ,CDCl₃) δ 188.39, 154.42, 148.22, 140.21. 134.28, 132.51, 131.30, 131.19, 128.92. 127.74, 127.72, 123.51, 117.80
(b) 2-(2,4-Dichlorphenoxy)-5-hydroxymethylanilin 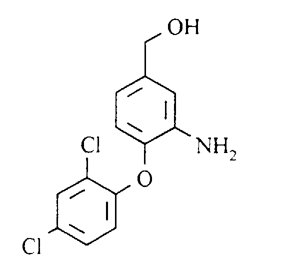 4-(2,4-Dichlorphenoxy)-3-nitrobenzaldehyd (15.0 g, 48 06 mmol) wurde in Dioxan (150ml) gelöst und eine Suspension von Raney Nickel in Wasser (10.0 g) wurde zugegeben. Die Mischung wurde bis zur Beendigung der Wasserstoffaufnahme bei 3.5 bis 4.0 bar hydriert. Danach wurde filtriert und vom Lösungsmittel befreit.
   Ausbeute: 13.5g = 99%
   ¹³C (100MHz, CDCl₃) δ 151.69, 142.15, 138.29, 130.34, 128.38, 127.94, 125.09, 119.30, 119.01, 117.26, 115.23, 64.91
(c) 2-(2.4-Dichlorphenoxy)-5-hydroxymethyl-N-methylsulfonylanilid 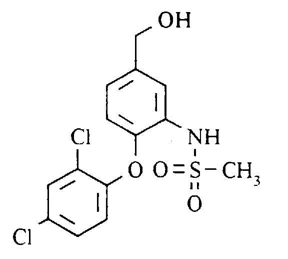 2-(2,4-Dichlorphenoxy)-5-hydroxymethylanilin (6.36 g, 22.38 mmol) wurde in Pyridin (50 ml) gelöst und bei -20°C wurde Methansulfonsäurechlorid (2.86 g, 25.0 mmol) zugetropft. Die Mischung kam über Nacht auf Raumtemperatur, anschließend wurde das Lösungsmittel abrotiert. Der Rückstand wurde in Ethylacetat gelöst und mit 1N HCI extrahiert. Die vereinigten organischen Phasen wurden über MgSO₄ getrocknet und nach dem Abdampfen des Lösungsmittels wurde der Rückstand chromatographisch gereinigt (CHCl₃/MeOH 19/l, Kieselgel).
   Ausbeute: 5.60 g = 69%
   ¹³C (100MHz, CDCl₃) δ 149.68, 146.49, 137.49, 130.92, 130.86, 128.52, 127.23, 126.92, 124.33, 122.10, 120.86, 116.16, 64.43, 39.87
(d) 2-(2,4-Dichlorphenoxy)-5-acetoxymethyl-4-nitro-N-methylsulfonylanilid 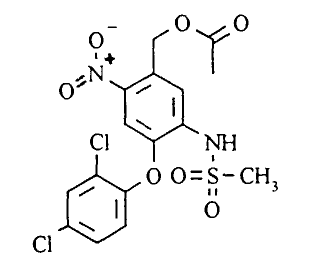 2-(2,4-Dichlorphenoxy)-5-hydroxymethyl-N-methylsulfonylanilid (1.00 g, 2.76mmol) wurde bei 110°C in Eisessig (10ml) gelöst und 65% HNO₃ (0.21 ml, 3.0 mmol) langsam zugetropft. Es wurde 2 h auf 110°C erhitzt. Nach dem Abkühlen wurde CHCl₃ (100 ml) zugegeben und mit NaHCO₃-Lösung extrahiert. Die organische Phase wurde über MgSO₄ getrocknet und das Lösungsmittel abrotiert. Der Rückstand wurde ohne Reinigung weiterverarbeitet.
   Ausbeute : 1.11 g = 96%
   ¹³C (100MHz, CDCl₃) δ 170.40, 147.99, 144.96, 142.35, 132.70, 132.43, 131.42, 129.64, 129.13, 127.42, 123.43, 117.81, 111.75, 62.67, 40.59, 20.71
(e) 2-(2,4-Dichlorphenoxy)-5-hydroxymethyl-4-nitro-N-methylsulfonylanilid 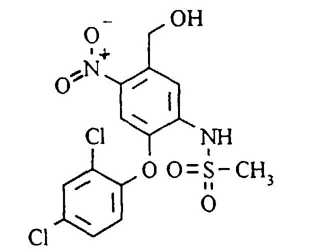 2-(2,4-Dichlorphenoxy)-5-acetoxymethyl-4-nitro-N-methylsulfonylanilid (1.05g, 2.49 mmol) wurde bei 0°C in Dioxan (20 ml), MeOH (20 ml) und Wasser (20 ml) gelöst und 2N NaOH (15 ml, 30 mmol) wurde zugegeben. Es wurde 3 h bei 0°C gerührt, mit 1N HCl angesäuert und mit Ethylacetat extrahiert. Die vereinigten organischen Phasen wurden über MgSO₄ getrocknet und das Lösungsmittel abrotiert. Der Rückstand wurde chromatographisch gereinigt (CHCl₃/MeOH 19/l, Kieselgel).
   Ausbeute : 0.88 g = 87%
   ¹³C (100MHz, CDCl₃) δ 150.10, 145.06, 141.71, 136.05, 134.31, 130.42, 129.52, 129,14, 125.95, 122.46, 120.54, 113.50, 59.91, 40.98
(f) 4-Amino-2-(2,4-dichlorphenoxy)-5-hydroxymethyl-N-methylsulfonylanilid 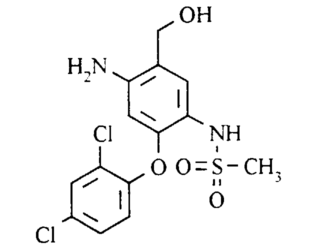 2-(2,4-Dichlorphenoxy)-5-hydroxymethyl-4-nitro-N-methylsulfonylanilid (0.88 g, 2.16 mmol) wurde in Dioxan (50 ml) gelöst und eine Suspension von Raney Nickel in Wasser (1.0 g) wurde zugegeben. Die Mischung wurde bis zur Beendigung der Wasserstoffaufnahme bei 3.5 bis 4.0 bar hydriert. Danach wurde filtriert und vom Lösungsmittel befreit. Der Rückstand wurde chromatographisch gereinigt (CHCl₃/MeOH 19/l, Kieselgel).
   Ausbeute: 0.70 g = 86%
   ¹³C (100MHz, CDCl₃) δ 150.31, 149.71, 146.23, 130.85, 130.71, 128.50, 128.05, 126.92, 122.08, 120.65, 115.85, 103.15, 63.53, 39.30
(g) 6-(2,4-Dichlorphenoxy)-5-methylsulfonylamino-2H-1,2-benzothiazolidin-1,1-dioxid
   4-Amino-2-(2,4-dichlorphenoxy)-5-hydroxymethyl-N-methylsulfonylanilid (0.70 g, 1.86 mmol) wurde bei 0°C in 8M Tetrafluoroborwasserstoffsäure (15 ml) suspendiert und eine Lösung von NaNO₂ (0.14 g, 2.0 mmol) in Wasser (1 ml) zugegeben. Es wurde 30 min gerührt und bei 0°C die Suspension in eine Mischung von ges. CuCl₂ Lösung in Wasser (10 ml) und ges. SO₂ Lösung in HOAc (50 ml) zugegeben . Nach 30 min bei dieser Temperatur wurde noch 30 min bei Raumtemperatur gerührt, mit Wasser verdünnt, mit Ethylacetat extrahiert, über MgSO₄ getrocknet und das Lösungsmittel abrotiert.
   Der Rückstand wurde in Dioxan (2 ml) gelöst und in eine Mischung von Dioxan (20 ml) und konz. Salmiak (20 ml) zugetropft. Die Lösung wurde 1 h bei 0°C gerührt und anschließend mit konz. HCl angesäuert. Es wurde mit Ethylacetat extrahiert, über MgSO₄ getrocknet und abrotiert. Der Rückstand wurde chromatographisch gereinigt (CH₂Cl₂/Ethylacetat 9/1)
   Ausbeute: 0.24g = 32%
   ¹³C (100MHz, CDCl₃) δ 148.31, 147.37, 142.82, 133.96, 132.37, 131.29, 131.19, 129.01, 127.49, 123.53, 112.91, 109.41, 78.52, 40.29

### Beispiel 3:

### 8-(2,4-Dichlorphenoxy)-7-methylsulfonylamino-3,4,5-dihydro-2-H-1,2-benzothiazepin-1,1-dioxid

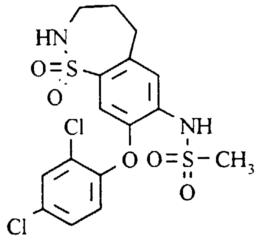

(a) 4-(2,4-Dichlorphenoxy)-3-nitrobenzaldehyd wurde wie in Beispiel 2 a) beschrieben hergestellt
(b) 4-(2,4-Dichlorphenoxy)-3-nitro-E-zimtsäure methylester 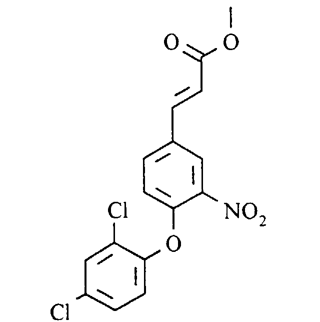 Trimethylphosphonoacetat (19.60 g, 107.62 mmol) wurde in THF (250 ml) gelöst und bei -70°C wurde n-BuLi (67.0 ml, 107.2 mmol) zugetropft und 15 min gerührt. Eine Lösung von 4-(2,4-Dichlorphenoxy)-3-nitrobenzaldehyd (29.0 g, 92.9 mmol) in THF (200 ml) wurde zugetropft und 1h bei -70°C gerührt. Danach wurde auf Phosphatpuffer (pH 7, 400 ml) gegossen, mit CH₂Cl₂ extrahiert, über MgSO₄ getrocknet und abrotiert. Der Rückstand wurde aus Ethanol umkristallisiert.
   Ausbeute: 31.3 g = 92%
   ¹³C (100MHz, CDCl₃) δ 165.56, 150.98, 149.15, 141.17, 133.19, 131.60, 131.01, 130.23, 128.63, 127.36, 125.20, 122.64, 119.98, 118.76, 51.93
(c) 4-(2,4-Dichlorphenoxy)-3-amino-phenylpropionsäure methylester 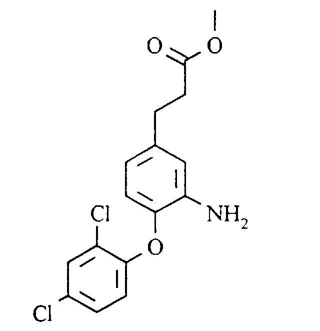 4-(2,4-Dichlorphenoxy)-3-nitro-E-zimtsäure methylester (31.30 g, 85.01 mmol) wurde in Dioxan (60 ml) gelöst und eine Suspension von Raney Nickel in Wasser (31.0 g) wurde zugegeben. Die Mischung wurde bis zur Beendigung der Wasserstoffaufnahme bei 3,5 bis 4.0 bar hydriert. Danach wurde filtriert und vom Lösungsmittel befreit.
   Ausbeute: 29.9 g = 99.9 %
   ¹³C (100MHz, CDCl₃) δ 173.30, 151.92, 141.05, 138.22, 137.99, 130.26, 128.07, 127.87, 124.85, 119.64, 118.48, 116.51, 51.59, 35.65, 30.47
(d) 4-(2,4-Dichlorphenoxy)-3-methylsulfonylamino-phenylpropionsäure methylester
   Die Synthese wurde analog zu Beispiel 2 (c) durchgeführt. Das Produkt wurde chromatographisch gereinigt (Petrolether/Ethylacetat 8/3, Kieselgel).
   ¹³C (100MHz, CDCl₃) δ 172.94, 149.90, 145.54, 137.17, 130.83, 130.57, 128.44, 127.30, 126.78, 125.60, 122.08, 121.83, 116.35, 51.66, 39.70, 35.52, 30.32
(e) 4-(2.4-Dichlorphenoxy)-3-methylsulfonylamino-6-nitro-phenylpropionsäure methylester
   Die Synthese wurde analog zu Beispiel 2 (d) durchgeführt. Das Produkt wurde chromatographisch gereinigt (CH₂Cl₂, Kieselgel).
   ¹³C (100MHz, CDCl₃) δ 172.63, 148.33, 144.38, 143.93, 132.80, 132.24, 132.04, 131.26, 129.00, 127.31, 123.19, 121.48, 111.99, 51.79, 40.52, 34.33, 28.56
(f) 1-[4-(2,4-Dichlorphenoxy)-3-methylsulfonylamino-6-amino-phenyl]-3-propanol 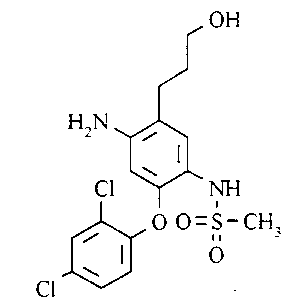 4-(2,4-Dichlorphenoxy)-3-methylsulfonylamino-6-nitro-phenylpropionsäure methylester (11.3 g, 24.4 mmol) wurde in THF (200 ml) gelöst und auf 0°C gelöst. LiAlH₄ (4.0 g, 105.3 mmol) wurde portionsweise zugegeben und die Mischung kam über Nacht auf Raumtemperatur. Es wurde angesäuert, mit Ethylacetat extrahiert, über MgSO₄ getrocknet und abrotiert. Da nur die Esterfunktion reduziert worden war, wurde der Rückstand noch wie in Beispiel 2(f) hydriert und chromatographisch gereinigt (CH₂Cl₂/MeOH 9/1, Kieselgel).
   Ausbeute: 5.84 g = 52%
   ¹³C (100MHz,DMSO-d₆) δ 151.46, 150.22, 146.71, 130.66, 129.80, 128.72, 127.85, 125.26, 121.67, 121.36, 114.83, 103,19, 60.28, 40,17, 31.66, 26.16
g) 8-(2,4-Dichlorphenoxy)-7-methylsulfonylamino-3,4,5-dihydro-2-H-1,2-benzothiazepin-1,1-dioxid
   Die Synthese wurde analog zu Beispiel 2 (g) durchgeführt. Der Rückstand nach der Amidierung wurde noch einmal in Toluol aufgenommen und 2 h unter Rückfluß erhitzt. Nach dem Abdampfen des Lösungsmittels wurde der Rückstand chromatographisch gereinigt (CH₂Cl₂, Kieselgel)
   ¹³C (100MHz,CDCl₃) δ 148.83, 145.24, 142.86, 135.40, 131.69, 131,13, 130.00, 128.82, 127.11, 122.78, 122,13, 113.52, 67.55, 40.24, 31.71, 29.17

### Beispiel 4:

### 6-(Phenoxy)-5-methylsulfonylamino-2H-1,2-benzothiazolidin-1,1-dioxid

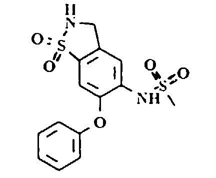

a) 4-Phenoxy-3-nitrobenzaldehyd 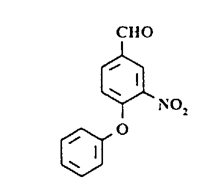 Die Synthese wurde analog zu Beispiel 2 (a) durchgeführt. Der Rückstand wurde aus Ethanol umkristallisiert.
b) 2-Phenoxy-5-hydroxymethylanilin 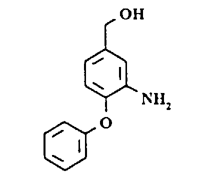 Die Synthese wurde analog zu Beispiel 2 (b) durchgeführt.
c) 5-Hydroxymethyl-2-phenoxy-N-methylsulfonylanilid 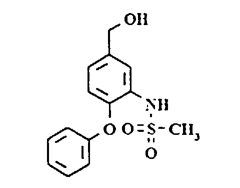 Die Synthese wurde analog zu Beispiel 2 ( c) durchgeführt.
d) 5-Hydroxymethyl-4-nitro-2-phenoxy-N-methylsulfonylanilid 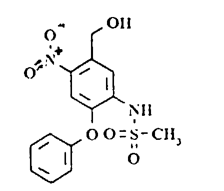 Die Synthese wurde analog zu Beispiel 2 (d) und 2 (e) durchgeführt.
e)4-Amino-5-hydroxymethyl-2-phenoxy-N-methylsulfonylanilid) 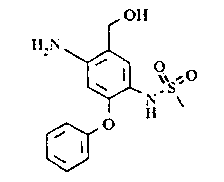 Die Synthese wurde analog zu Beispiel 2 (f) durchgeführt.
f) 6-(Phenoxy)-5-methylsulfonylamino-2H-1,2-benzothiazolidin-1,1-dioxid
   Die Synthese wurde analog zu Beispiel 2 (g) durchgeführt.
   ¹³C (100MHz , CDCl₃) δ 159.63, 147.85, 143,17, 134.67, 133.77, 130.64, 129.46, 124.41, 120.68, 113.95, 75.20, 41.04

### Beispiel 5:

### 3-(2,4-Difluorphenoxy)-4-methylsulfonylaminobenzolsulfonamid

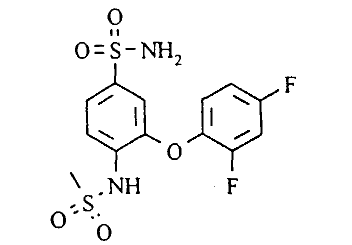

(a) 2-(2,4-Difluorphenoxy)-nitrobenzol
   Die Synthese wurde analog zu Beispiel 1 (a) durchgeführt. Zur Reinigung wurde mit CH₂Cl₂/Petrolether 1/9 über Kieselgel chromatographiert.
   ¹³C (100MHz, CDCl₃) δ 160.87, 160.77, 158.41, 158.31, 155.40, 155.27, 152.88, 152.76, 150.71, 140.37, 138.59, 138.56, 138.51, 138.48, 134.18, 125.92, 123.17, 123.05, 122.96, 117.93, 111.95, 111.91, 111.72, 111.68, 106.05, 105.84, 105.78, 105.57
(b) 2-(2,4-Difluorphenoxy)-anilin
   Die Synthese wurde analog zu Beispiel 1(b) durchgeführt.
   ¹H (400MHz. CDCl₃) δ 159.52, 159.42, 157.09, 156.98, 154.88, 154.76, 152.38, 152.26, 144.06, 140.73, 140.70, 140.62, 140.58, 137.71, 124.60, 120.90, 120.88, 120.81, 120.79, 118.66, 117.55, 116.48, 111.22, 111.18, 111.00, 110.95, 105.55, 105.33, 105.28, 105.06
(c) 2-(2,4-Difluorphenoxy)-N-methylsulfonylanilid
   Die Synthese wurde analog zu Beispiel 1 (c) durchgeführt. Das Produkt wurde ohne Reinigung für die weitere Synthese eingesetzt.
   ¹³C (100MHz, CDCl₃) δ 160.82, 160.72, 158.36, 158.22, 155.58, 155.47, 153.08, 152.96, 148.18, 138.52, 138.48, 138.40, 138.36, 129.72, 126.80, 125.81, 124.01, 123.37, 123.36, 123.28, 123.26, 122.27, 114.92, 111.97, 111.93, 111.74, 111.70, 106.12, 105.91, 105.86, 105.64, 39.45
(d) 3-(2,4-Difluorphenoxy)-4-methylsulfonylaminobenzolsulfonamid
   Chlorsulfonsäure (3.6 ml, 53.6 mmol) wurde in Chloroform (40 ml) gelöst und auf 0°C gekühlt. Eine Lösung von 2-(2,4-Difluorphenoxy)-N-methylsulfonyl-anilid (4.02 g, 13.4 mmol) in Chloroform (20 ml) wurde zugetropft und die Lösung 30 min, bei 0°C und 2 h bei Raumtemeratur gerührt. Danach wurde Phosphorpentachlorid (11 g. 53.6 mmol) zugegeben und 2 h nachgerührt. Das nicht umgesetzte Phosphorpentachlorid wurde abfiltriert und das Filtrat wurde mit Eiswasser extrahiert, über MgSO₄ getrocknet und abrotiert. Der Rückstand (7.1 g) wurde in Dioxan (70 ml) gelöst und bei 10°C Ammoniak eingeleitet. Nach 1.5 h wurde Ethylacetat (100 ml) und mit IN HCl extrahiert. Die organische Phase wurde über MgSO₄ getrocknet und abrotiert. Der Rückstand wurde chromatographisch gereinigt (Kieselgel, CH₂Cl₂/Ethylacetat 19/1)
   Ausbeute: 3.9 g= 57%
   ¹³C (100MHz, DMSO-d₆) δ 160.21, 160.10, 157.78, 157.67, 155.08, 154.95, 152.59, 152.46,148.84, 141.02,138.51, 138.47, 138.40, 138.36, 130.79, 123.90, 123.84, 121.15, 112.87,112.67, 112.64, 112.45, l12.41,106.35, 106.13, 106.07, 105.85, 40.88

### Beispiel 6:

### 3-(2,4-Difluorthiophenoxy)-4-methylsulfonylaminobenzolsulfonamid

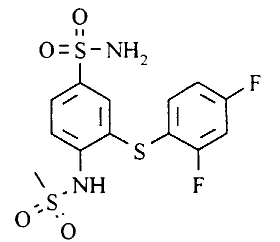

(a) 2-(2,4-Difluorthiophenoxy)-nitrobenzol
   Die Synthese wurde analog zu Beispiel 1 (a) durchgeführt. Zur Reinigung wurde aus Petrolether kristallisiert.
   ¹³C (100MHz, CDCl₃) δ 166,16, 166.05, 165.06, 164.93, 163.63, 163.52, 162.54, 162.42, 145.18, 139.01, 139.00, 138.91, 137.22, 133.69, 127.38, 126.05, 125.44, 114.06, 114.02, 113.87, 113.83, 113.27, 113.23, 113.05, 113.01, 105.78, 105.52, 105.26
(b) 2-(2,4-Difluorthiophenoxy)-anilin
   Die Synthese wurde analog zu Beispiel 1(b) durchgeführt.
   ¹³C (100MHz, CDCl₃) δ 162.90, 162.80, 161,15, 161.03, 160.44, 160.33, 158.70, 158.58, 148.88, 137.31, 131.35, 130.37, 130.34, 130.28, 130.25, 118.92, 115.46, 113.22, 112.04, 112.01, 111.83, 111.79, 104.46, 104.02, 103.95,
(c) 2-(2,4-Difluorthiophenoxy)-N-methylsulfonylanilid
   Die Synthese wurde analog zu Beispiel 1 (c) durchgeführt. Das Produkt wurde ohne Reinigung für die weitere Synthese eingesetzt.
   ¹³C (100MHz, CDCl₃) δ 164.25, 164.13, 162.42, 162.30, 161.75, 161.62, 160.42, 160.30, 138.63, 136.09, 133.27, 133.16, 131.09, 125.37, 119.82, 112.62, 112.59, 112.41, 112.37, 105.27, 105.01, 104.75, 39.70
(d) 3-(2,4-Difluorthiophenoxy)-4-methylsulfonylaminobenzolsulfonamid
   Die Synthese wurde analog zu Beispiel 1 (d) und 1 (e) durchgeführt. Der Rückstand wurde aus Ethanol umkristallisiert.
   ¹³C (100MHz, DMSO-d₆) δ 164.61, 164.50, 163.22, 163.09, 162.13, 162.01, 160.75, 160.62, 142.33, 138.26, 136.95, 136.86, 132.32, 126.95, 126.38, 125.97, 125.30, 115.18, 115.14, 115.00, 114.96, 113.50, 113.47, 113.29, 113.25, 105.86, 105.59, 105.33, 41.33

### Beispiel 7:

### 3-(2,4-Dichlorthiophenoxy)-4-methylsulfonylaminobenzolsulfonamid

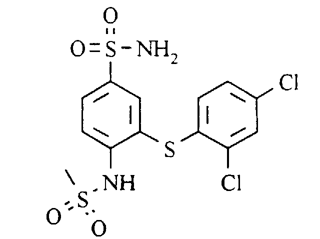

(a) 2-(2,4-Dichlorthiophenoxy)-nitrobenzol
   Die Synthese wurde analog zu Beispiel 1 (a) durchgeführt. Zur Reinigung wurde aus Petrolether kristallisiert.
   ¹³C (100MHz, CDCl₃) δ 145.45, 139.39, 138.85, 136.44, 134.88, 134.54, 130.59, 129.22, 128.72, 128.42, 126.94, 126.14
(b) 2-(2,4-Dichlorthiophenoxy)-anilin
   Die Synthese wurde analog zu Beispiel 1(b) durchgeführt.
   ¹³C (100MHz, CDCl₃) δ 149.19, 137.84, 134.88, 131.98, 131.58, 131.16, 129.26, 127.45, 127.23, 119.09, 115.54, 112.13
(c) 2-(2,4-Dichlorthiophenoxy)-N-methylsulfonylanilid
   Die Synthese wurde analog zu Beispiel 1 (c) durchgeführt. Der Rückstand wurde chromatographisch gereinigt (CH₂Cl₂/Kieselgel).
   ¹³C (100MHz, CDCl₃) δ 139.70, 137.54, 133.33, 132.93, 132.15, 129.89, 128.53, 127.88, 125.50. 119.63, 119.55, 39.88
(d) 3-(2,4-Dichlorthiophenoxy)-4-methylsulfonylaminobenzolsulfonamid
   Die Synthese wurde analog zu Beispiel 1 (d) und 1 (e) durchgeführt. Der Rückstand wurde aus CH₂Cl₂/MeOH 24/1 kristallisiert.
   ¹³C (100MHz, DMSO-d₆) δ 141.78, 141.06, 134.07, 132.82, 132.10, 131.04, 129.72, 128.58, 127.26, 127.25, 124.93, 41.26

### Beispiel 8:

### 3-(2-Chlor-4-fluorthiophenoxy)-4-methylsulfonylaminobenzol-sulfonamid

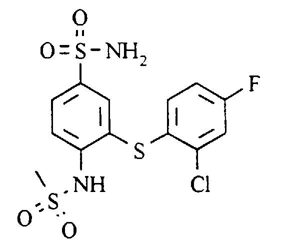

(a) 2-(2-Chlor-4-fluorthiophenoxy)-nitrobenzol
   Die Synthese wurde analog zu Beispiel 1 (a) durchgeführt. Zur Reinigung wurde aus Diethylether kristallisiert.
   ¹³C (100MHz, CDCl₃) δ 165.10, 162.56, 145.17, 141.60, 141.49, 139.54, 139.45, 137.08, 133.68, 127.49, 126.04, 125.89, 125.85, 125.44, 118.74, 118.49, 115.87, 115.65
(b) 2-(2-Chlor-4-fluorthiophenoxy)-anilin
   Die Synthese wurde analog zu Beispiel 1(b) durchgeführt.
   ¹³C (100MHz, CDCl₃) δ 161.79, 159.33, 149.11, 137.72, 131.77, 131.36, 131.33, 127.91, 127.83, 119.04, 117.20, 116.94, 115.50, 114.79, 114.58, 112.90
(c) 2-(2-Chlor-4-fluorthiophenoxy)-N-methylsulfonylanilid
   Die Synthese wurde analog zu Beispiel 1 (c) durchgeführt. Der Rückstand wurde aus Ethanol umkristallisiert.
   ¹³C (100MHz, CDCl₃) δ 162.71, 160.22, 155.57, 139.30, 136.99, 133.91, 133.80, 131.74, 130.12, 130.03, 129.74, 129.70, 125.50, 120.88, 119.77, 117.90, 117.65, 115.30, 115.08, 39.82
(d) 3-(2-Chlor-4-fluorthiophenoxy)-4-methylsulfonylaminobenzolsulfonamid
   Die Synthese wurde analog zu Beispiel 1 (d) und 1 (e) durchgeführt.
   ¹³C (100MHz, DMSO-d₆) δ 163.76, 161.27, 143.05, 137.92, 137.81, 137.28, 137.19, 135.07, 129.49, 126.94, 124.45, 118.46, 118.21, 116.34, 116.12, 41.27

### Beispiel 9:

### 3-(2.4-Dibromphenoxy)-4-methylsulfonylaminobenzolsulfonamid

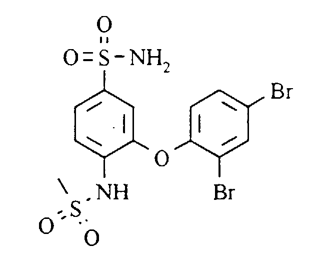

(a) 2-(2,4-Dibromphenoxy)-nitrobenzol
   Die Synthese wurde analog zu Beispiel 1 (a) durchgeführt. Zur Reinigung wurde aus CH₂Cl₂/Petroleteher 1/1.5 kristallisiert.
   ¹³C (100MHz, CDCl₃) δ 151.84, 149.60, 141.05, 136.46, 134.33, 131.99, 126.03, 123.88, 121.82, 119.60, 118.09, 115.94
(b) 2-(2.4-Dibromphenoxy)-anilin
   Die Synthese wurde analog zu Beispiel 1(b) durchgeführt.
   ¹³C (100MHz, CDCl₃) δ 153.42, 142.48, 138.37, 135.84, 131.53, 125.61, 119.83, 118.89, 118.81, 116.73, 11548, 114.01
(c) 2-(2,4-Dibromphenoxy)-N-methylsulfonylanilid
   Die Synthese wurde analog zu Beispiel 1 (c) durchgeführt. Der Rückstand wurde chromatographisch gereinigt (CH₂Cl₂ /Kieselgel) und anschließend aus Ethanol umkristallisiert.
   ¹³C (100MHz, CDCl₃) δ 151.42, 146.96, 136.49, 132.12, 127.44, 125.84, 124.59, 122.31, 122.15, 118.60, 118.16, 116.43, 116.05, 39.81
(d) 3-(2.4-Dibromphenoxy)-4-methylsulfonylaminobenzolsulfonamid
   Die Synthese wurde analog zu Beispiel 1 (d) und 1 (e) durchgeführt. Das Produkt wurde chromatographisch gereinigt (Kieselgel; CH₂Cl₂/MeOH 50/1).
   ¹³C (100MHz, DMSO-d₆) δ 151.67, 147.90, 141.03, 135.77, 132.68, 131.47, 123.99, 123.24, 121.57, 117.54, 116.01, 114.20, 41.05

### Beispiel 10:

### 3-(Cyclohexyloxy)-4-methylsulfonylaminobenzolsulfonamid

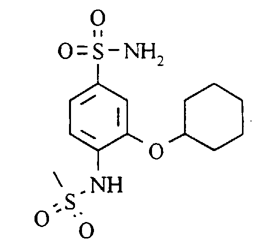

(a) 2-(Cyclohexyloxy)-nitrobenzol
   Cyclohexanol (5 ml, 47 mmol) und NaH (2.0 g, 50 mmol) wurden in Dioxan (80 ml) 1 h auf 70°C erhitzt. Nach dem Abkühlen auf Raumtemperatur wurde eine Lösung von 1-Fluor-2-nitrobenzol (7.0 g, 49.6 mmol) in Dioxan (20 ml) zugesetzt und über Nacht bei Raumtemperatur gerührt. Die Mischung wurde auf Wasser gegossen und mit CH₂Cl₂ extrahiert. Es wurde über MgSO₄ getrocknet und das Lösungsmittel abgedampft. Der Rückstand chromatographisch gereinigt (CH₂Cl₂/Petrolether 6/4)
   Ausbeute: 3.9 g = 37%
   ¹³C (100MHz, CDCl₃) δ 150.96, 141.05, 133.29, 125.18, 119.73, 116.00, 66.85, 31.05, 25.20, 22.87
(b) 2-(Cyclohexyloxy)-anilin
   Die Synthese wurde analog zu Beispiel 1(b) durchgeführt.
   ¹³C (100MHz, CDCl₃) δ 145.22, 137.62, 121.23, 118.28, 115.41, 114.12, 76.09, 32.13, 25.74, 23.84
(c) 2-(Cyclohexyloxy)-N-methylsulfonylanilid
   Die Synthese wurde analog zu Beispiel 1 (c) durchgeführt. Der Rückstand wurde aus Ethanol umkristallisiert.
   ¹³C (100MHz, CDCl₃) δ 147.59, 126.93, 125.45, 121.21, 121.10, 113.06, 76.63, 39.07, 31.92, 25,40, 23.87
(d) 3-(Cyclohexyloxy)-4-methylsulfonylaminobenzolsulfonamid
   Chlorsulfonsäure (0.15 ml, 2.26 mmol) wurde in Chloroform (6 ml) gelöst und auf -25°C gekühlt. Bei dieser Temperatur wurde langsam eine Lösung von 2-(Cyclohexyloxy)-N-methylsulfonylanilid (0.50 g, 1.85 mmol) in Chloroform (5 ml) zugetropft. Nach 1 h bei -25°C wurde noch einmal Chlorsulfonsäure (0.15 ml. 2.26 mmol) zugetropft und eine weitere Stunde nachgerührt. Anschließend wurde Phosphorpentachlorid (0.9 g. 4.5 mmol) zugesetzt und weitere 2 h bei -20°C gerührt. Es wurde auf Eiswasser gegossen, die organische Phase abgetrennt. über MgSO₄ getrocknet und das Lösungsmittel abrotiert. Der Rückstand wurde ohne weitere Reinigung eingesetzt.
   Die Amidierung zum Sulfonamid wurde wie in Beispiel 1 (e) beschrieben durchgeführt. Das Rohprodukt wurde chromatographisch gereinigt (Kieselgel, Petrolether/Ethylacetat 4/6). Ausbeute: 0.28 g = 43 %
   ¹³C (100MHz, DMSO-d₆) δ 152.94, 135.78, 126.47, 124.53, 123.47, 113.29, 75.89, 40.59, 31.04, 25,17, 23.22

### Beispiel 11:

### 3-(2-Chlor-4-bromphenoxy)-4-methylsulfonylaminobenzol-sulfonsäure-N-ethyl-amid

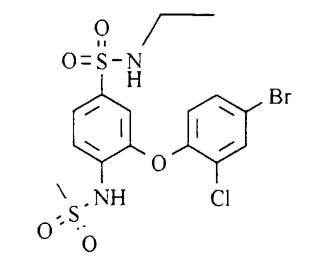

(a) 2-(2-Chlor-4-bromphenoxy)-nitrobenzol
   Die Synthese wurde analog zu Beispiel 1 (a) durchgeführt. Das Produkt konnte ohne Reinigung weiter eingesetzt werden.
   ¹³C (100MHz, CDCl₃) δ 150.59, 149.68, 140.91, 134.30, 133.66, 131.32, 127.22, 126.03, 123.81, 122.07, 119.37, 117.87
(b) 2-(2-Chlor-4-bromphenoxy)-anilin
   Die Synthese wurde analog zu Beispiel 1(b) durchgeführt.
   ¹³C (100MHz, CDCl₃) δ 152.34, 142.52, 138.31, 133.08, 130.85, 125.53, 125.30, 119.60, 119.22, 118.81, 116.71, 115.20
(c) 2-(2-Chlor-4-bromphenoxy)-N-methylsulfonylanilid
   Die Synthese wurde analog zu Beispiel 1 (c) durchgeführt. Das Produkt konnte ohne Reinigung weiterverarbeitet werden.
   ¹³C (100MHz, CDCl₃) δ 150.21, 147.07, 133.71, 131.45, 127.34, 127.24, 125.84, 124.52, 122.48, 122.31, 117.94, 116.17, 39.73
(d) 3-(2-Chlor-4-bromphenoxy)-4-methylsulfonylaminobenzolsulfonsäure N-ethylamid
   Die Synthese wurde analog zu Beispiel 1 (d) und 1 (e) durchgeführt. Das Produkt wurde chromatographisch gereinigt (Kieselgel, CH₂Cl₂/Ethylacetat 8/2)
   ¹³C (100MHz, DMSO-d₆) δ 150.35, 147.72, 137.18, 133.13, 132.14, 132.04, 126.55, 123.58, 123.35, 122.62, 117.34, 114.62, 41.00, 37.57, 14.73

### Beispiel 12:

### 3-(2-Brom-4-chlorphenoxy)-4-methylsulfonylaminobenzol-sulfonsäure N-ethyl-amid

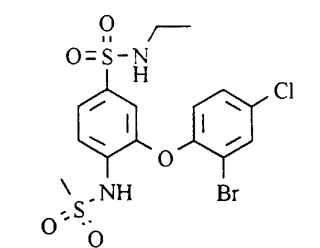

(a) 2-(2-Brom-4-chlorphenoxy)-nitrobenzol
   Die Synthese wurde analog zu Beispiel 1 (a) durchgeführt. Das Produkt konnte ohne Reinigung weiter eingesetzt werden.
   ¹³C (100MHz, CDCl₃) δ 151.27. 149.74, 140.90, 134.28, 133.72, 130.91, 129.04, 126.02, 123,77, 121,51, 119.43, 115.64
(b) 2-(2-Brom-4-chlorphenoxy)-anilin
   Die Synthese wurde analog zu Beispiel 1(b) durchgeführt
   ¹³C (100MHz, CDCl₃) 152.88, 142.63, 138.34, 133.13, 128.59, 128.45, 125.52, 119.69, 118.80, 118.51, 116.71, 113.66
(c) 2-(2-Brom-4-chlorphenoxy)-N-methylsulfonylanilid
   Die Synthese wurde analog zu Beispiel 1 (c) durchgeführt. Das Produkt konnte ohne Reinigung weiterverarbeitet werden.
   ¹³C (100MHz, CDCl₃) δ 150.87, 147.07, 133.74, 130.98, 129.17, 127.39, 125.82, 124.53, 122.28, 121.80, 116.32, 115.72, 39.80
(d) 3-(2-Brom-4-chlorphenoxy)-4-methylsulfonylaminobenzolsulfonsäure N-ethylamid
   Die Synthese wurde analog zu Beispiel 1 (d) und 1 (e) durchgeführt. Das Produkt wurde chromatographisch gereinigt (Kieselgel, CH₂Cl₂/Ethylacetat 8/2)
   ¹³C (100MHz, DMSO-d₆) δ 151.07, 147.92, 137.16, 133.20, 131.87, 129.81, 123.62, 123.02, 122.47, 120.20, 115.74, 114.48, 41.07, 37.57, 14.75

### Beispiel 13:

### 3-(3-Aminosulfonyl-5-chlorthienyl-2-thio)-4-methylsulfonylamino-benzolsulfonamid

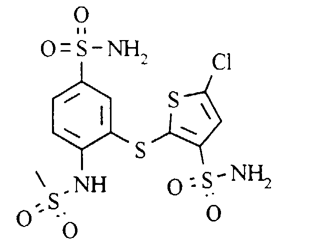

(a) 2-(Thienyl-2-thio)-nitrobenzol
   Die Synthese wurde analog zu Beispiel 1 (a) durchgeführt. Das Produkt wurde chromatographisch gereinigt (Kieselgel/Petrolether/Ethylacetat 4/6)
   ¹³C (100MHz, CDCl₃) δ 144.90, 139.91, 138.40, 133.77, 133.38, 128.76, 128.71, 127.62, 125.64, 125.32
(b) 2-(5-Chlorthienyl-2-thio)-nitrobenzol
   2-(Thienyl-2-thio)-nitrobenzol (7.77 g, 32.7 mmol) wurde in MeCN (50 ml) gelöst und unter Stickstoff auf 60°C erwärmt, N-Chlorsuccinimid (4.65 g. 35.0 mmol) wurde rasch zugegeben und 1 h unter Rückfluß erhitzt. Das Lösungsmittel wurde im Vakuum abgedampft, der Rückstand in CH₂Cl₂ gelöst, 2 mal mit 4N NaOH extrahiert und über MgSO4 getrocknet. Das Lösungmittel wurde abgedampft und der Rückstand ohne weitere Reinigung eingesetzt. Ausbeute: 8.56g=85%
   ¹³C (100MHz, CDCl₃) 144.94, 139.02, 138.37, 136.37, 133.96, 127.91, 127.53, 127,50, 125.76, 125.71
(c) 2-(5-Chlorthienyl-2-thio)-anilin
   Die Synthese wurde analog zu Beispiel 1(b) durchgeführt. Das Produkt wurde ohne Reinigung weiterverarbeitet.
   ¹³C (100MHz, CDCl₃) δ 147.42, 135.11, 131.40, 130.98, 130.75, 129.30, 126.61, 118.92, 115.65, 115.16
(d) 2-(5-Chlorthienyl-2-thio)-N-methylsulfonylanilid
   Die Synthese wurde analog zu Beispiel 1 (c) durchgeführt. Das Produkt wurde chromatographisch gereinigt (Kieselgel, CH₂Cl₂)
   ¹³C (100MHz, CDCl₃) δ 136.86, 133.66, 133.60, 130.51, 130.31, 127.09, 126,14, 125.79, 125.36, 120.98, 39.79
(e) 3-(3-Aminosulfonyl-5-chlorthienyl-2-thio)-4-methylsulfonylaminobenzol-sulfonamid
   Die Synthese wurde analog zu Beispiel 1 (d) und (e) durchgeführt. Das Produkt wurde durch Kristallisation aus CH₂Cl₂ gereinigt.
   ¹³C (100MHz, DMSO-d₆) δ 141.92, 141.66, 141.07, 136.04, 131.89, 128.62, 128.21, 127.91, 127.38, 124.45, 41.09

### Beispiel 14:

### 3-(3,5-Dichlorthienyl-2-thio)-4-methylsulfonylaminobenzol-sulfonamid

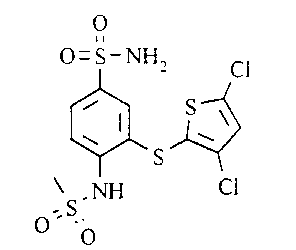

(a) 2-(Thienyl-2-thio)-nitrobenzol
   Die Synthese wurde analog zu Beispiel 1 (a) durchgeführt. Das Produkt wurde chromatographisch gereinigt (Kieselgel/Petrolether/Ethylacetat 4/6)
   ¹³C (100MHz, CDCl₃) δ 144.90, 139.91, 138.40, 133.77, 133.38, 128.76, 128.71, 127.62, 125.64, 125.32
(b) 2-(3,5-Dichlorthienyl-2-thio)-nitrobenzol
   2-(Thienyl-2-thio)-nitrobenzol (1.00 g, 3.68 mmol) wurde in MeCN (20 ml) gelöst und unter Stickstoff auf 60°C erwärmt. N-Chlorsuccinimid (4.91 g, 36 8 mmol) wurde rasch zugegeben und 4 h unter Rückfluß erhitzt. Das Lösungsmittel wurde im Vakuum abgedampft, der Rückstand in CH₂Cl₂ gelöst, 4 mal mit 4N NaOH extrahiert und über MgSO₄ getrocknet. Das Lösungmittel wurde abgedampft und der Rückstand aus CH₂Cl₂ kristallisiert
   Ausbeute: 1.06 g = 94% ¹³C (100MHz, CDCl₃) 144.98, 136.73, 135.48. 134.60, 134.21, 128.18, 127.10, 126.08, 126.04, 121.93
(c) 2-(3,5-Dichlorthienyl-2-thio)-anilin
   Die Synthese wurde analog zu Beispiel 1(b) durchgeführt. Das Produkt wurde ohne Reinigung weiterverarbeitet.
   ¹³C (100MHz, CDCl₃) δ 147.85, 135.90, 131.23, 130.82, 128.18, 126.93, 126.58, 118.88, 116.17, 115.67
(d) 2-(3.5-Dichlorthienyl-2-thio)-N-methylsulfonylanilid
   Die Synthese wurde analog zu Beispiel 1 (c) durchgeführt. Das Produkt wurde aus Petrolether/Ethylacetat kristallisiert.
   ¹³C (100MHz, CDCl₃) δ 137.43, 134.58, 133.16, 130.95, 130.09, 127.37, 125.68, 124.89, 124.40, 120.67, 39.86
(e) 2-(3,5-Dichlorthienyl-2-thio)-N-methylsulfonylaminobenzolsulfonamid
   Die Synthese wurde analog zu Beispiel 1 (d) und (e) durchgeführt. Das Produkt wurde durch Kristallisation aus Aceton/CH₂Cl₂ gereinigt.
   ¹³C (100MHz, DMSO-d₆) δ 143.02, 137.26, 134.27, 133.14, 132.42, 128.84, 127.45, 125.29, 125.20, 122.67, 41.26

### Beispiel 15:

### 3-(2,4-Dimethyl-6-aminosulfonylphenoxy)-4-methylsulfonyl-aminobenzol-sulfon-amid

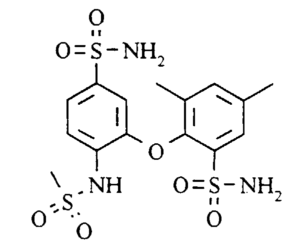

(a) 2-(2.4-Dimethylphenoxy)-nitrobenzol
   Die Synthese wurde analog zu Beispiel 1 (a) durchgeführt. Zur Reinigung wurde chromatographiert (Kieselgel; CH₂Cl₂/Petroleteher 7/3).
   ¹³C (100MHz, CDCl₃) δ 151.61, 150.62, 140.85, 135.00, 133.94, 132.43, 129.84, 127.94, 125.72, 121.77, 120.05, 117.86, 20.74, 15.87
(b) 2-(2,4-Dimethylphenoxy)-anilin
   Die Synthese wurde analog zu Beispiel 1(b) durchgeführt.
   ¹³C (100MHz, CDCl₃) δ 152.49, 144.77, 137.58, 132.88, 132.01, 128.72, 127.53, 123.47, 118.63, 118.06, 117.40, 116.09, 20.64, 16.01
(c) 2-(2-4-Dimethylphenoxy)-N-mcthylsulfonylanilid
   Die Synthese wurde analog zu Beispiel 1 (c) durchgeführt. Der Rückstand wurde aus Ethanol umkristallisiert.
   ¹³C (100MHz, CDCl₃) δ 150.79, 148.31, 134.77, 132.45, 129.51, 128.02, 126.91, 125.62, 123.01, 121.51, 119.73, 115.51, 39.43, 20.72, 15.96
(d) 3-(2.4-Dimethyl-6-aminosulfonylphenoxy)-4-methylsulfonylamino-benzolsul-fonamid
   Die Synthese wurde analog zu Beispiel 1 (d) und 1 (e) durchgeführt. Das Produkt wurde chromatographisch gereinigt (Kieselgel:CH₂Cl₂/Ethylacetat 3/2).
   ¹³C (100MHz, DMSO-d₆) δ 150.26, 148.71, 141.40, 141.03, 135.54, 134.06, 132.55, 130.87, 123.83, 120.79, 119.00, 112.97, 40,91, 19.16, 15.63

### Beispiel 16:

### 3-(5-Methyl-1,3,4-thiadiazolyl-2-thio)-4-methylsulfonylamino-benzolsulfonamid

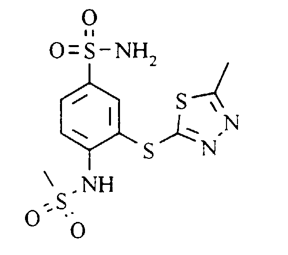

(a) 2-(5-Methyl-1,3,4-thiadiazolyl-2-thio)-nitrobenzol
   Die Synthese wurde analog zu Beispiel 1 (a) durchgeführt. Zur Reinigung wurde chromatographiert (Kieselgel: CH₂Cl₂).
   ¹³C (100MHz, CDCl₃) δ 170.57, 160.37, 146.09, 134.13, 133.29, 129.55, 127.37, 125.80, 16.08
(b) 2-(5-Methyl-1,3,4-thiadiazolyl-2-thio)-anilin
   Die Synthese wurde analog zu Beispiel 1(b) durchgeführt.
   ¹³C (100MHz, CDCl₃) δ 168.84, 165.95, 149.03, 136.98, 132,74, 118.96, 115.99, 113.04, 15.65
(c) 2-(5-Methyl-1,3,4-thiadiazolyl-2-thio)-N-methylsulfonylanilid
   Die Synthese wurde analog zu Beispiel 1 (c) durchgeführt. Der Rückstand wurde aus CH₂Cl₂ umkristallisiert.
   ¹³C (100MHz, CDCl₃) δ 167.04, 164.39, 140.04, 136.97, 132.86, 125.52, 120.86, 119.96, 40.12, 15.76
(d) 3-(5-Methyl-1,3,4-thiadiazolyl-2-thio)-4-methylsulfonylaminobenzolsulfonamid
   Die Synthese wurde analog zu Beispiel 1 (d) und 1 (e) durchgeführt. Das Produkt wurde aus CH₂Cl₂ kristallisiert.
   ¹³C (100MHz, DMSO-d₆) δ 167.62, 164.43, 141.82, 141.45, 132.62, 128.54, 126.14, 124.93, 40.94, 15.49

### Beispiel 17:

### 3-(4-Chlorphenylthio)-4-methylsulfonylaminobenzolsulfonamid

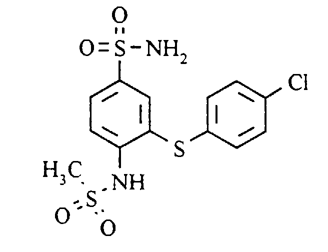

(a) 3-Chlorsulfanilamid
   Sulfanilamid (50.0 g; 290.34 mmol) wurde in MeCN (500 ml) gelöst und auf 60°C erwärmt. N-Chlorsuccinimid (40.06 g; 300 mmol) wurde rasch zugegeben und es wurde 2 h unter Rückfluß erhitzt. Nach dem Abkühlen wurde das Lösungsmittel abrotiert, der Rückstand in Ethylacetat gelöst und 2 mal mit 4N Ammoniak-Lösung extrahiert. Die organische Phase wurde mit Wasser gewaschen. über MgSO₄ getrocknet und eingedampft. Es wurde aus Ethylacetat umkristallisiert.
   Ausbeute: 45 g = 75%
   ¹³C (100MHz, DMSO-d₆) δ 147.87, 131.46, 127.22, 125.91, 115.84, 114.27
(b) 3-Chlor-4-nitrobenzolsulfonamid
   3-Chlorsulfanilamid (2.27 g, 11 mmol) wurde in einer Mischung aus Eisessig (50 ml) und 35%igem Wasserstoffperoxid (18 ml) gelöst und auf 70°C erwärmt. Nach 3 h wurde der kristalline Niederschlag abfiltriert und das Filtrat mit Ethylacetat verdünnt. Zur Vernichtung der überschüssigen Peroxide wurde Eisen(II)sulfat zugegeben und nach dem Abfiltrieren das Lösungsmittel abgedampft. Der Rückstand wurde in Ethylacetat gelöst, mit Wasser gewaschen. über MgSO₄ getrocknet und eingedampft.
   Ausbeute: 1.35 g = 52 %
   ¹³C (100MHz, DMSO-d₆) δ 149.26, 148.45, 128.72, 126.91, 125.98, 125.91
(c) 3-(4-Chlorphenylthio)-4-nitro-benzolsulfonamid
   3-Chlor-4-nitrobenzolsulfonamid (1.08 g; 4.53 mmol), 4-Chlorthiophenol (0.66 g; 4.53 mmol) und K₂CO₃ (0.64 g: 4.60 mmol) wurden in Dioxan (30 ml) 3 h unter Rückfluß erwärmt. Danach wurde mit CH₂Cl₂ verdünnt, filtriert und mit Wasser gewaschen. Die organische Phase wurde über MgSO₄ getrocknet und eingedampft. Das Produkt wurde aus Petrolether/Ethylacetat umkristallisiert.
   Ausbeute: 1.0 g = 64%
   ¹³C (100MHz, DMSO-d₆) δ 148.57. 146.43, 138.07, 137.08, 135.76, 130.70, 128.74, 127.27, 125.43, 123.52
(d) 4-Amino-3-(4-chlorphenylthio)-benzolsulfonamid
   3-(4-Chlorphenylthio)-4-nitro-benzolsulfonamid (0.50 g, 1.45 mmol), NH₄Cl (0.16 g, 2.90 mmol) und Fe-Pulver (0.40 g: 7.25 mmol) wurden in EtOH (10 ml)/Wasser (5 ml) suspendiert und 30 min unter Rückfluß erhitzt. Die Lösung wurde filtriert, abrotiert und der Rückstand in CH₂Cl₂ aufgenommen. Es wurde mit Wasser gewaschen, über MgSO₄ getrocknet und eingedampft. Der Rückstand wurde roh weiter eingesetzt
   Ausbeute: 0.43 g = 94%
   ¹³C (100MHz, CDCl₃)d 152.17, 136.12, 133.59, 132.24, 130.75, 129.86, 129.38, 128.37, 114.69, 114,09
(e) 3-(4-Chlorphenylthio)-4-methylsulfonylaminobenzolsulfonamid
   4-Amino-3-(4-chlorphenylthio)-benzolsulfonamid (0.43 g: 1.37 mmol) wurde in Pyridin (10 ml) gelöst und bei 0°C wurde Methansulfonsäurechlorid (4.7 g; 41.0 mmol) zugetropft. Die
   Lösung kam über Nacht auf Raumtemperatur, wurde auf Wasser gegossen und mit CH₂Cl₂ extrahiert. Das Lösungsmittel wurde abrotiert und der Rückstand in Dioxan (10 ml)/ 2N wässriger NaOH (10 ml) gerührt. Nach dem Ansäuem mit 2N HCl wurde mit Ethylacetat extrahiert, über MgSO₄ getrocknet und eingedampft. Der Rückstand wurde aus CH₂Cl₂ umkristallisiert. Ausbeute: 0.28 g = 52%
   ¹³C (100MHz, DMSO-d₆) δ 141.50, 140.23, 133.03, 132.82, 132.73, 129.79, 129.78, 129.46, 126.16, 123.90, 41.03

### Beispiel 18:

### 3-(N-Methylimidazolyl-2-thio)-4-methylsulfonylamino-benzol-sulfonamid

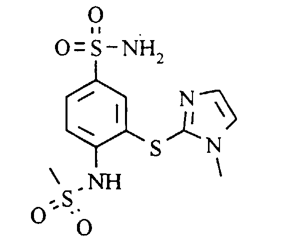

(a) 3-(N-Methylimidazolyl-2-thio)-4-nitro-benzolsulfonamid
   Die Synthese wurde analog zu Beispiel 17 (c) durchgeführt. Das Produkt wurde aus CH₂Cl₂ umkristallisiert.
   ¹³C (100MHz, DMSO-d₆) δ 148.98, 145.94, 137.18, 133.80, 130.95, 127.69, 126.47, 124.49, 123.95, 33.64
(b) 3-(N-Methylimidazolyl-2-thio)-4-amino-benzolsulfonamid
   Die Synthese wurde analog zu Beispiel 17 (d) durchgeführt. Das Produkt wurde aus Ethylacetat umkristallisiert.
   ¹³C (100MHz, DMSO-d₆) δ 152.15, 136.60, 132.13, 131.33, 128.99, 128.01, 124.84, 114.40, 112.40, 33.66
(c) 3-(N-Methylimidazolyl-2-thio)-4-methylsulfonylamino-benzolsulfonamid
   Die Synthese wurde analog zu Beispiel 17 (e) durchgeführt. Das Produkt wurde aus CH₂Cl₂ /MeOH umkristallisiert.
   ¹³C (100MHz, DMSO-d₆) δ 141,72, 139.05, 135.74, 131.13, 129.85, 127.15, 125,79, 125,70, 125.48, 41,19, 33.63

### Beispiel 19:

### 3-(Cyclohexylthio)-4-methylsulfonylaminobenzolsulfonamid

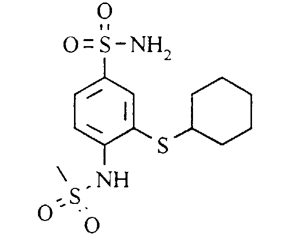

(a) 3-(Cyclohexylthio)-4-nitro-benzolsulfonamid
   Die Synthese wurde analog zu Beispiel 17 (c) durchgeführt. Das Produkt wurde aus Diisopropylether umkristallisiert.
   ¹³C (100MHz, DMSO-d₆) δ 148.82, 148.10, 135.04, 126.68, 126.12, 122.87, 44.15, 32.07, 25.30, 25.18
(b) 3-(Cyclohexylthio)-4-amino-benzolsulfonamid
   Die Synthese wurde analog zu Beispiel 17 (d) durchgeführt. Das Produkt wurde aus Diisopropylether umkristallisiert.
   ¹³C (100MHz, CDCl₃) δ 152.79, 135.75, 130.02, 128.49, 116.79, 113.84, 47,70, 33.61, 26.01, 25.61
(c) 3-(Cyclohexylthio)-4-methylsulfonylamino-benzolsulfonamid
   Die Synthese wurde analog zu Beispiel 17 (e) durchgeführt.
   ¹³C (100MHz, CDCl₃) δ 143.35, 137.04, 134.83, 128.44, 123.18, 116.86, 49.57, 40.49, 33.41, 25,91, 25.35

### Beispiel 20:

### 3-(5-Trifluoromethylpyridyl-2-thio)-4-methylsulfonylamino-benzol-sulfonamid

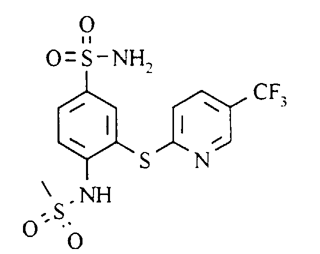

(a) 2-(5-Trifluormethylpyridyl-2-thio)-nitrobenzol
   Die Synthese wurde analog zu Beispiel 1 (a) durchgeführt. Zur Reinigung wurde der Rückstand mit Petrolether ausgerührt.
   ¹³C (100MHz, CDCl₃) δ 161.90, 146.99, 146.96, 146.92, 146.88, 144.82, 135.05, 134.08, 134.05, 134.02, 133.99, 133.05, 129.14, 127.87, 125.37, 124.68, 124.38, 123.69, 122.00
(b) 2-(5-Trifluormethylpyridyl-2-thio)-anilin
   Die Synthese wurde analog zu Beispiel 1(b) durchgeführt.
   ¹³C (100MHz, CDCl₃) δ 165.60, 149.24, 146.54, 146.49, 146.45, 146.40, 137.71, 133.60, 133.60, 133.57, 133.54, 133.50, 132.31, 129.26, 125.03, 122.90, 122.57, 122.32, 122.24, 119.35, 119.13, 115.69, 118.54, 111.41
(c) 2-(5-Trifluormethylpyridyl-2-thio)-N-methylsulfonylanilid
   Die Synthese wurde analog zu Beispiel 1 (c) durchgeführt. Der Rückstand wurde chromatographisch gereinigt (Kieselgel; CH₂Cl₂/Ethylacetat 19/1).
   ¹³C (100MHz, CDCl₃) δ 163.03, 163.02, 146.90, 146.86, 146.81, 146.77, 140.46, 137.76, 134.17, 134.13, 134.10, 134.07, 132.53, 125.59, 124.68, 124.56, 124.23, 123.90, 123.57, 121.02, 120.87, 119.29, 40.02
(d) 3-(5-Trifluormethylpyridyl-2-thio)-4-methylsulfonylaminobenzolsulfonamid
   Die Synthese wurde analog zu Beispiel 1 (d) und 1 (e) durchgeführt.
   ¹³C (100MHz, DMSO-d₆) δ 165.38, 146.27, 140.11, 137.79, 134.32, 131.63, 126.93, 125.67, 123.65, 121.88, 121.56, 120.80, 40.68

### Beispiel 21:

### 3-(4-Methoxyphenylthio)-4-methylsulfonylaminobenzolsulfonamid

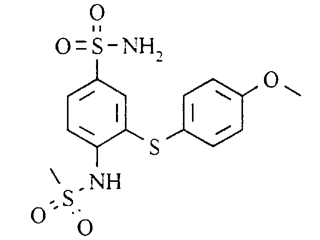

(a) 3-(4-Methoxyphenylthio)-4-nitro-benzolsulfonamid
   Die Synthese wurde analog zu Beispiel 17 (c) durchgeführt. Das Produkt wurde aus CH₂Cl₂ umkristallisiert.
   ¹³C (100MHz, DMSO-d₆) δ 161.28, 148.48, 145.73, 140.12, 137.54, 127.21, 124.78, 122.89, 119.37, 116.31, 55.61
(b) 3-(4-Methoxyphenylthio)-4-amino-benzolsulfonamid
   Die Synthese wurde analog zu Beispiel 17 (d) durchgeführt. Das Produkt wurde aus CH₂Cl₂/Petrolether umkristallisiert.
   ¹³C (100MHz, DMSO-d₆) δ 158.66, 152.24, 133.68, 131.23, 130.91, 128.14, 125.36, 115.16, 114.44, 113.92, 55.37
(c) 3-(4-Methoxyphenylthio)-4-methylsulfonylarmino-benzolsulfonamid
   Die Synthese wurde analog zu Beispiel 17 (e) durchgeführt. Das Produkt wurde chromatographisch gereinigt (Kieselgel, CH₂Cl₂/MeOH 50/1)
   ¹³C (100MHz, DMSO-d₆) δ 160.20, 142.05, 137.43, 135.63, 135.20, 126.53, 126.00, 124.48, 121.77, 115.82, 55.49, 41.44

### Beispiel 22:

### 3-(4-Methoxyphenylamino)-4-methylsulfonylamino-benzolsulfonamid

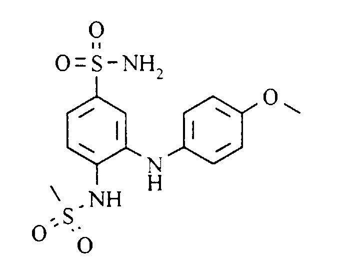

(a) 3-(4-Methoxyphenylamino)-4-nitro-benzolsulfonamid
   Natriumhydrid (1.69 g; 42.26 mmol) wurde in absolutem DMF suspendiert und bei 0°C wurde eine Lösung von p-Anisidin (5.20 g; 42.26 mmol) zugetropft. Nach 30 min wurde 3-Chlorsulfanilamid (1.00 g; 4.23 mmol) zugegeben und bei 40°C für 30 min gerührt. Die Mischung wurde auf Wasser gegossen, mit konz. Salzsäure auf pH 1-2 eingestellt und mit Ethylacetat extrahiert. Die vereinigten organischen Phasen wurden über MgSO₄ getrocknet . Der Rückstand wurde chromatographisch gereinigt (Kieselgel; CH₂Cl₂).
   ¹³C (100MHz, DMSO-d₆) δ 157.83, 150.90, 137.80, 131.44, 130.66, 128.86, 125.82, 124.33, 115.13, 111.50, 55.56
(b) 3-(4-Methoxyphenylamino)-4-amino-benzolsulfonamid
   Die Synthese wurde analog zu Beispiel 1 (b) durchgeführt. Das Produkt wurde chromatographisch gereinigt (Kieselgel; CH₂Cl₂).
   ¹³C (100MHz, DMSO-d₆) δ 154.36, 137.89, 137.22, 137.01, 120,11, 119.70, 119.60, 118.96, 117.04, 114.91, 55.72
(c) 3-(4-Methoxyphenylamino)-4-methylsulfonylamino-benzolsulfonamid
   Die Synthese wurde analog zu Beispiel 17 (e) durchgeführt. Das Produkt wurde chromatographisch gereinigt (Kieselgel, CH₂Cl₂/MeOH 10/1)
   ¹³C (100MHz, DMSO-d₆) δ 154.99, 145.93, 134.76, 131.77, 130.93, 123.31, 122.21, 114.83, 114.07, 113.30, 55.37, 40.64

### Beispiel 23: 3-(2,4-Dimethylphenoxy)-4-methysulfonylaminobenzolsulfonamid

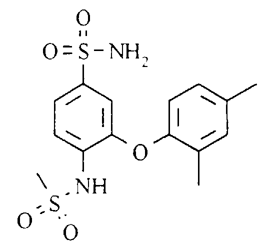

(a) 3-(2.4-Dimethylphenoxy)-4-nitro-benzolsulfonamid
   Die Synthese wurde analog zu Beispiel 17 (c) durchgeführt. Das Produkt wurde chromatographisch gereinigt (Kieselgel:CH₂Cl₂/MeOH 25/1).
   ¹³C (100MHz, DMSO-d₆) δ 150.41, 149.51, 149.01, 141.41, 135.57, 132.68, 129.34, 128.59, 126.70, 120.50, 119.75, 114.22, 20.46, 15.43
(b) 3-(2,4-Dimethylphenoxy)-4-amino-benzolsulfonamid
   Die Synthese wurde analog zu Beispiel 17 (d) durchgeführt. Das Produkt wurde aus CH₂Cl₂ umkristallisiert.
   ¹³C (100MHz, DMSO-d₆) δ 151.64, 142.65, 133.16, 132,14, 130.59, 128.76, 127.85, 121.80, 118.94, 113.72 113.55, 20.39, 15.74
(c) 3-(2,4-Dimethylphenoxy)-4-methylsulfonylamino-benzolsulfonamid
   Die Synthese wurde analog zu Beispiel 17 (e) durchgeführt. Das Produkt wurde chromatographisch gereinigt (Kieselgel. CH₂Cl₂/Ethylacetat 9/1)
   ¹³C (100MHz, DMSO-d₆) δ 150.32. 149.63. 141.03, 134.57, 132.37, 130.39, 129.70, 128.30, 123.98, 120.54, 119.98, 112.30, 40.95, 20.47, 15.73

### Beispiel 24:

### 3-(Pyridyl-3-oxy)-4-methylsulfonylaminobenzolsulfonamid

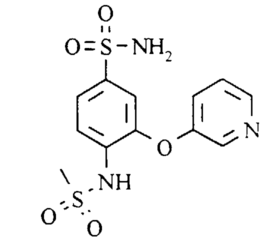

(a) 2-(Pyridyl-3-oxy)-nitrobcnzol
   Die Synthese wurde analog zu Beispiel 1 (a) durchgeführt. Das Produkt wurde chromatographisch gereinigt (Kieselgel, Ethylacetat/Petrolether 1:1)
   ¹³C NMR (100 MHz, CDCl₃) (152.91, 149.41, 145.48, 141.08, 134.48, 125.93, 125.59, 124.47, 124.31, 121.14.
(b) 2-(Pyridyl-3-oxy)-anilin
   Zu einer stark gerührten Suspension bestehend aus Rh/C und 2-(Pyridyl-3-oxy)nitrobenzol in THF (25 mL), wurde N₂H₄·H₂O langsam bei 0 °C zugetropft. Die Reaktionslösung ließ man langsam über Nacht auf Raumtemperatur kommen. Das Reaktionsgemisch wurde filtriert und das Filtrat eingedampft. Der so entstandene Rückstand wurde in Ethylacetat (100 ml) aufgenommen und mit verdünnter Salzsäure (3 x 50 ml, pH 2) gewaschen. Die wäßrige Lösung wurde neutralisiert (NaHCO₃) und mit CH₂Cl₂ (3 x 50 ml) extrahiert. Die organische Lösung wurde über MgSO₄ getrocknet und anschließend eingedampft. Das erhaltene Öl wurde aus Ethylacetat umkristallisiert
   Ausbeute: 5.60 g = 64%
   ¹³C NMR (100 MHz, CDCl₃) (154.06, 143.83, 142.20, 140.20, 138.89, 125.66, 124.01, 123.58, 120.16, 118.76, 116.72.
(c) 2-(Pyridyl-3-oxy)-N-methylsulfonylanilid
   Die Synthese wurde analog zu Beispiel 1 (c) durchgeführt. Das Produkt wurde chromatographisch gereinigt (Kieselgel, CH₂Cl₂/MeOH 10:1)
   ¹³C NMR (100 MHz, CDCl₃) (152.66, 146.54, 145.51, 141.26, 128.44, 125.72, 125.67, 125.09, 124.37, 121.71, 118.09, 39.84.
(d) 3-(Pyridyl-3-oxy)-4-methylsulfonylaminobenzolsulfonamid
   Die Synthese wurde analog zu Beispiel 1 (d) und 1 (e) durchgeführt Der Rückstand wurde mit Diisopropylether digeriert und aus Ethanol umkristallisiert
   ¹³C (100MHz, DMSO-d₆) (152.50, 147.68, 145.42, 141.42, 140.90, 132.30, 126.37, 124.92, 123,68, 121.97, 115.81, 40.94

### Beispiel 25: 3-(2-Chlorphenylthio)-4-methylsulfonylaminobenzolsulfonamid

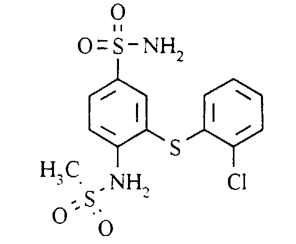

(a) 3-(2-Chlorphenylthio)-4-nitro-benzolsulfonamid
   Die Synthese wurde analog zu Beispiel 17 (c) durchgeführt. Das Produkt wurde aus Petrolether/CH₂Cl₂ umkristallisiert.
   ¹³C (100MHz, DMSO-d₆) δ 148.74, 146.51, 138.38, 137.96, 136.51, 133.00, 131.19, 129.24, 128.43, 127.50, 124.99, 123.75
(b) 3-(2-Chlorphenylthio)-4-amino-benzolsulfonamid
   Die Synthese wurde analog zu Beispiel 17 (d) durchgeführt. Das Produkt wurde aus CH₂Cl₂ umkristallisiert.
   ¹³C (100MHz, DMSO-d₆) δ 153.62. 135.57, 134.97, 131.54, 130.27. 129.79. 129.52, 127.90, 12692, 126.22, 114.42, 108.95
(c) 3-(2-Chlorphenylthio)-4-methylsulfonylamino-benzolsulfonamid
   Die Synthese wurde analog zu Beispiel 17 (e) durchgeführt. Das Produkt wurde chromatographisch gereinigt (Kieselgel, CH₂Cl₂/MeOH 19/1) und aus CHCl₃ umkristallisiert
   ¹³C (100MHz. DMSO-d₆) δ 141.48, 141.08, 133.36, 133.28, 131.23, 130.80. 130.28, 129.17, 128.45, 127.60, 126.99, 124.62, 41.22

### Beispiel 26:

### 3-(4-Bromphenylthio)-4-methylsulfonylaminobenzolsulfonamid

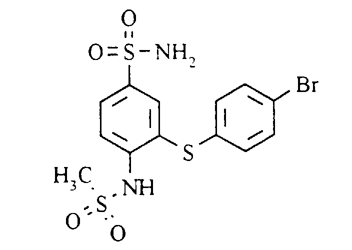

(a) 3-(4-Bromphenylthio)-4-nitro-benzolsulfonamid
   Die Synthese wurde analog zu Beispiel 17 (c) durchgeführt. Das Produkt wurde aus Petrolether/CH₂Cl₂ umkristallisiert.
   ¹³C (100MHz DMSO-d₆) δ 148.56, 146.50, 137.85, 137.19, 133.62, 129.27, 127.26, 125.50, 124.49, 123.56
(b) 3-(4-Bromphenylthio)- 4-amino-benzolsulfonamid
   Die Synthese wurde analog zu Beispiel 17 (d) durchgeführt.
   ¹³C (100MHz, CDCl₃)d 153.16, 135.54, 135.18, 132.08, 131.34, 129.20, 128.72, 118.83, 114.28, 110.70
(c) 3-(4-Bromphenylthio)-4-methylsulfonylaminobenzolsulfonamid
   Die Synthese wurde analog zu Beispiel 17 (e) durchgeführt. Das Produkt wurde chromatographisch gereinigt (Kieselgel ,Petrolether/Ethylacetat 1/1).
   ¹³C (100MHz, DMSO-d₆) δ 141.75, 139.98, 133.44, 132.79, 132.75, 130.11, 130.05, 126.41, 125.04, 121.25, 41.21

### Beispiel 27:

### 3-(4-Trifluoromethoxyphenylthio)-4-methylsulfonylaminobenzol-sulfonamid

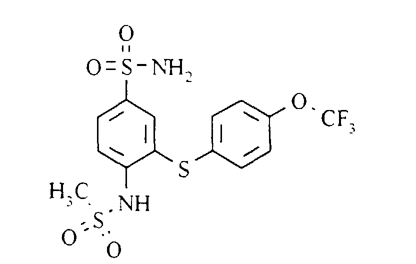

¹³C (100MHz, DMSO-*d*_{*6*}) δ 141.70, 141.53, 141.06, 132.48, 128.91, 127.72, 127.42, 127.10, 126.47, 126.40, 126.36,124.38, 41.10

### Beispiel 28: 3-(Furyl-2-thio)-4-methylsulfonylaminobenzolsulfonamid

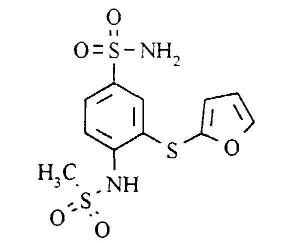

### Beispiel 29: 3-(3-Chlorphenylthio)-4-methylsulfonylaminobenzolsulfonamid

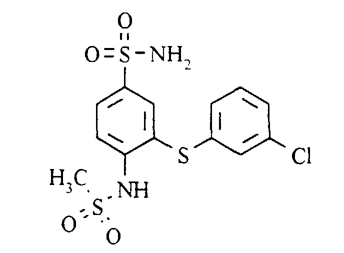

(a) 3-(3-Chlorphenylthio)-4-nitro-benzolsulfonamid 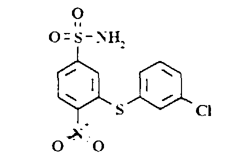 Die Synthese wurde analog zu Beispiel 14 (c ) durchgeführt. Das Produkt wurde aus Petrolether/CH₂Cl₂ umkristallisiert.
   ¹³C (100MHz, DMSO-d₆) δ 148.58, 146.62, 137.58, 134.73, 134.37, 133.85, 132.22. 132.12, 130.70, 127.23, 125.74, 123.70
(b) 3-(3-Chlorphenylthio)-4-amino-benzolsulfonamid 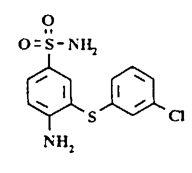 Die Synthese wurde analog zu Beispiel 14 (d) durchgeführt. Das Produkt wurde aus CHCl₃ umkristallisiert.
   ¹³C (100MHz, DMSO-d₆) δ 153.32, 138.54, 135.44, 133.87, 131.36, 13092, 129.43, 125.83, 125.71, 125.16, 114.33, 110.09
(c) 3-(3-Chlorphenylthio)-4-methylsulfonylamino-benzolsulfonamid 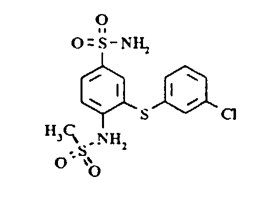 Die Synthese wurde analog zu Beispiel 14 (e) durchgeführt. Das Produkt wurde chromatographisch gereinigt ( Kieselgel, CH₂Cl₂/MeOH 19/1).
   ¹³C (100MHz, DMSO-d₆) δ 141.62, 140.60, 136.64, 134,16, 131.42, 131.05, 129.46, 128.80, 128.74, 127.72, 126.93, 124.73, 41.19

### Beispiel 30: 3-(2,4,6-Trichlorphenylthio)-4-methylsulfonylaminobenzol-sulfonamid

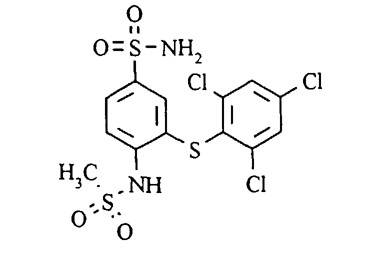

¹³C (100MHz, DMSO-*d*₆) δ 143,01, 141.54, 137.05, 136.34, 134.17, 129.56, 129.34, 128.76, 127.84, 126.39, 124.43, 123.00, 41.49

### Beispiel 31: 3-(4-Trifluoromethylphenylthio)-4-methylsulfonylaminobenzol-sulfonamid

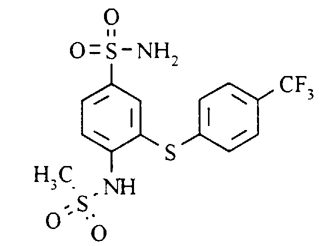

### Beispiel 32: 3-(Phenylthio)-4-methylsulfonylaminobenzolsulfonamid

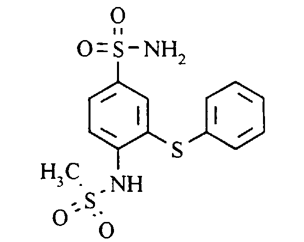

### Beispiel 33: 3-(2-Brom-4-chlorphenylthio)-4-methylsulfonylaminobenzolsulf-onamid

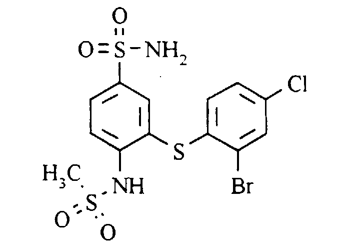

### Beispiel 34: 3-(2,5-Dichlorphenylthio)-4-methylsulfonylaminobenzolsulfonamid

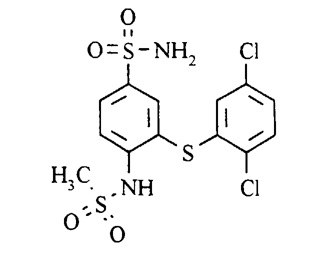

¹³C (100MHz, DMSO-*d*_{*6*}) d 141.76, 141.64, 136.21, 132.70, 131.96, 131.60, 131.28, 129.08, 128.54, 127.85, 125.95, 124.81, 41.29

### Beispiel 35: 3-(2,3-Dichlorphenylthio)-4-methylsulfonylaminobenzolsulfonamid

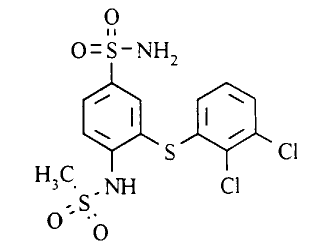

¹³C (100MHz, DMSO-*d*_{*6*}) δ 142.07, 141.49, 137.28, 132.70, 132.57, 129.74, 128.99, 128.65, 128.02, 127.84, 125.37, 124.24, 41.19

### Beispiel 36:

### 3-(2,4-Dichlorphenoxy)-4-methylsulfonylaminobenzolsulfonsäure N-methylamid

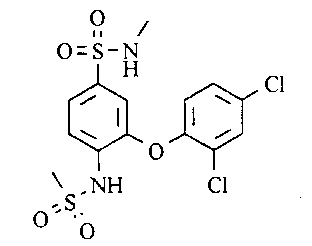

3-(2,4-Dichlorphenoxy)-4-methylsulfonylaminobenzolsulfonsäure N-methyl-amid (HN-56203) 3-(2,4-Dichlorphenoxy)-4-methylsulfonylaminobenzolsulfonsäurechlorid (0.56g, g 1.3 mmol) wurde in Dioxan (15 ml) gelöst und bei 0°C zu einer Lösung von Methylamin Hydrochlorid (1.18 g, 17 mmol) in 1N wässriger NaOH (15 ml, 15 mmol) und Dioxan (20 ml) zugetropft. Die Mischung wurde 1 h bei 0°C gerührt, mit IN HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, CH₂Cl₂/Ethylacetat 9/1).
Ausbeute: 0.25 g = 45%
¹³C (100MHz, CDCl₃) δ 148.35, 146.21, 134.97, 132.03, 131.28, 131.16, 128.98, 127,15, 123.15, 123.12, 119.56, 113.81, 40.45, 29.18

### Beispiel 37:

### 3-(2,4-Dichlorphenoxy)-4-methylsulfonylaminobenzolsulfonsaure N,N-dimethyl-amid

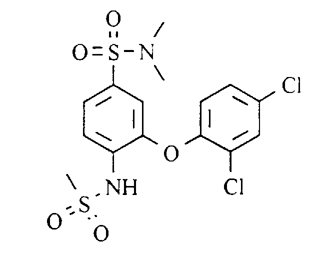

3-(2,4-Dichlorphenoxy)-4-methylsulfonylaminobenzolsulfonsäurechlorid (1.12g, 2.6 mmol) wurde in Dioxan (5 ml) gelöst und bei 0°C zu einer Lösung von Dimethylamin Hydrochlorid (1.10 g. 13.4 mmol) in 1N wässriger NaOH (13 ml, 13 mmol) und Dioxan (13 ml) zugetropft. Die Mischung wurde 1 h bei 0°C gerührt mit IN HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, Petrolether/Ethylacetat 6/4).
Ausbeute: 0.27g=24%
¹³C (100MHz, DMSO-d₆) δ 150.02, 147.24, 133.01, 13081, 130.42, 129.53, 129.18, 126.03, 123.74, 123.17, 122.57, 115.72, 41.03, 37.59

### Beispiel 38:

### 3-(2,4-Dichlorphenoxy)-4-methylsulfonylaminobenzolsulfonsäure azetidinium-amid

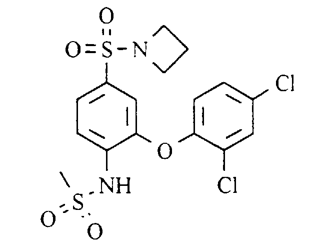

3-(2,4-Dichlorphenoxy)-4-methylsulfonylaminobenzolsulfonsäurechlorid (0.51g, 1.2 mmol) wurde in Dioxan (5 ml) gelöst und bei 0°C zu einer Lösung von Azetidinium Tetrafluoroborat (1.48 g, 10.2 mmol) in IN wässriger NaOH (10 ml, 10 mmol) und Dioxan (20 ml) zugetropft. Die Mischung wurde 1 h bei 0°C gerührt, mit IN HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, CH₂Cl₂/Ethylacetat 9/l),
Ausbeute: 0.18 g = 33%
¹³C (100MHz, DMSO-d₆) δ 149.94, 147.28, 133.32, 130.49, 129.70, 129.58, 129.26, 126.15, 124.39, 122.87, 122.86, 116.02, 50.94, 41.08, 14.87

### Beispiel 39: 3-(2,4-Dichlorphenoxy)-4-methylsulfonylaminobenzolsulfonsäure N-ethylamid

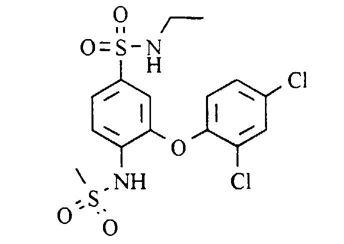

3-(2.4-Dichlorphenoxy)-4-methylsulfonylaminobenzolsulfonsäurechlorid (1.64g, 3.8 mmol) wurde in Dioxan (5 ml) gelöst und bei 0°C zu einer Lösung von Ethylamin Hydrochlorid (2.5 l g. 30.8 mmol) in IN wässriger NaOH (30 ml. 30 mmol) und Dioxan (30 ml) zugetropft. Die Mischung wurde 1 h bei 0°C gerührt, mit IN HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, CH₂Cl₂/Ethylacetat 10/0.5).
Ausbeute: 0.46 g = 28%
¹³C (100MHz, DMSO-d₆) δ 149.88, 147.82, 137.13, 131.96, 130.44, 129.66, 129.24, 126.31, 123.58, 123.03, 122.58, 114.53, 41.00, 37.57, 14.73

### Beispiel 40:

### 3-(2,4-Dichlorphenoxy)-4-methylsulfonylaminobenzolsulfonsäure N,N-diethyl-amid

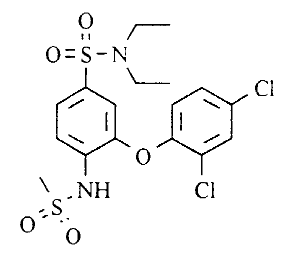

3-(2,4-Dichlorphenoxy)-4-methylsulfonylaminobenzolsulfonsäurechlorid (1.08 g. 2.50 mmol) wurde in Dichlormethan (5 ml) gelöst und bei 0°C zu einer Lösung von Diethylamin (0.30 ml, 2.51 mmol) in Pyridin (20 ml) zugetropft. Die Mischung wurde 1 h bei 0°C gerührt, mit IN HCl angesäuert und mit Ethylacetat extrahiert. Die vereinigten organischen Phasen wurden über MgSO₄ getrocknet und das Lösungsmittel wurde abrotiert Der Rückstand wurde chromatographisch gereinigt Kieselgel CH₂Cl₂).
Ausbeute: 0.22 g = 19%
¹³C (100MHz, DMSO-d₆) δ 149.98, 147.50, 136.21, 132.37, 130.43, 129.63, 129.21, 126.15, 123.56, 122.86, 122.72, 114.90, 41.77, 41.02, 14.02

### Beispiel 41:

3-(2,4-Dichlorphenoxy)-4-methylsulfonylaminobenzolsulfonsäure morpholinamid 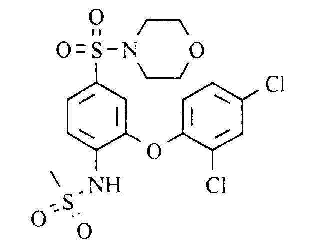 3-(2,4-Dichlorphenoxy)-4-methylsulfonylaminobenzolsulfonsäurechlorid (1.36g, 3.2 mmol) wurde in Dioxan (5 ml) gelöst und bei 0°C zu einer Lösung von Morpholin (2.25 ml, 25.6 mmol) in IN wässriger NaOH (26 ml,26 mmol) und Dioxan (20 ml) zugetropft. Die Mischung wurde 1 h bei 0°C gerührt, mit IN HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, CH₂Cl₂/Ethylacetat 9/1).
Ausbeute: 0.93 g = 60%
¹³C (100MHz, DMSO-d₆) δ 150.02, 147.15, 133.44, 130.43, 129.51, 129.31, 129.18, 126.23, 125.98, 123.95, 123.02, 122,51, 116.78, 115,83, 65.41, 45.83, 41.03

### Beispiel 42: 3-(2,4-Dichlorphenoxy)-4-methylsulfonylaminobenzolsulfonsäure Hydroxyamid

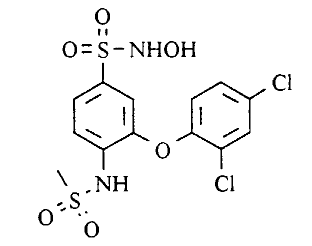

3-(2,4-Dichlorphenoxy)-4-methylsulfonylaminobenzolsulfonsäurechlorid (0.17g, 0.40 mmol) wurde in Dioxan (3 ml) gelöst und bei 0°C zu einer Lösung von Hydroxylamin Hydrochlorid (0.28 g, 4.0 mmol) und Natriumcarbonat (0.43 g, 4.0 mmol) in Wasser (5 ml) und Dioxan (2 ml) zugetropft. Die Mischung wurde 2 h bei 0°C gerührt, mit conc. HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, Chloroform/MeOH 9/1).
Ausbeute: 0.1 g = 62%
¹³C (100MHz, DMSO-d₆) δ 149.92, 147.35, 133.60, 132.91. 130.40, 129.60, 129.20, 126.25, 124.41, 122.99, 122.90, 116.22, 41.09

### Beispiel 43: 3-(2,4-Dichlorphenoxy)-4-methylsulfonylaminobenzolsulfonsäure O-methylhydroxyamid

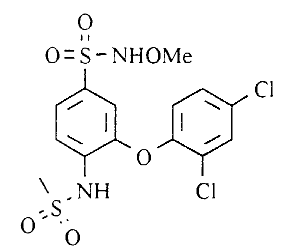

3-(2,4-Dichlorphenoxy)-4-methylsulfonylaminobenzolsulfonsäurechlorid (1.1g, 2.55 mmol) wurde in CH₂Cl₂ (5 ml) gelöst und bei 0°C zu einer Lösung von O-Methylhydroxylamin Hydrochlorid (0.3 g, 3.6 mmol) und DMAP (0.44 g, 3.6 mmol) in Pyridin (20 ml) zugetropft. Die Mischung wurde 2 h bei 0°C gerührt und dann abrotiert. Der Rückstand wurde im Vakuum getrocknet und chromatographisch gereinigt (Kieselgel, Chloroform/MeOH 50/l).
Ausbeute: 0.28 g = 25%
¹³C (100MHz, DMSO-d₆) δ 149.89, 147.25, 133.47, 133.06, 130.42, 129.64, 129.20, 126.24, 124.42, 122.85, 122.71, 116.00, 64.44, 41.11

### Beispiel 44: 3-(2,4-Dichlorphenoxy)-4-methylsulfonylaminobenzolsulfonsäure N.O-dimethylhydroxyamid

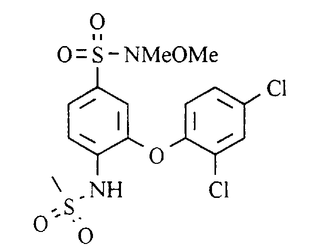

3-(2,4-Dichlorphenoxy)-4-methylsulfonylaminobenzolsulfonsäurechlorid (1.1g, 2.55 mmol) wurde in CH₂Cl₂ (5 ml) gelöst und bei 0°C zu einer Lösung von N,O-Dimethylhydroxylamin Hydrochlorid (0.34 g. 3.5 mmol) und DMAP (0.44 g, 3.6 mmol) in Pyridin (20 ml) zugetropft. Die Mischung wurde 2 h bei 0°C gerührt und dann abrotiert. Der Rückstand wurde im Vakuum getrocknet und chromatographisch gereinigt (Kieselgel, CH₂Cl₂).
Ausbeute: 0.43 g = 37%
¹³C (100MHz, DMSO-d₆) δ 149.94, 146.71, 134.37, 130.45, 129.64, 129.18, 126.97, 126.10, 125.77, 122.77, 122.20, 117.43, 63.36, 41.12, 38.97

### Beispiel 45:

### 3-(2,4-Dichlorphenoxy)-4-methylsulfonylaminobenzolsulfonsäure N-benzylamid

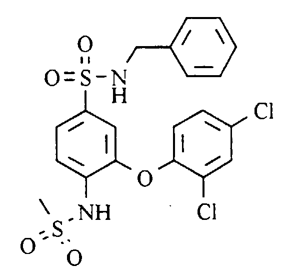

3-(2,4-Dichlorphenoxy)-4-methylsulfonylaminobenzolsulfonsäurechlorid (1.08g, 2.51 mmol) wurde in CH₂Cl₂ (5 ml) gelöst und bei 0°C zu einer Lösung von Benzylamin (0.35 ml, 3.2 mmol) in Pyridin (20 ml) zugetropft. Die Mischung wurde 2 h bei 0°C gerührt und dann abrotiert. Der Rückstand wurde im Vakuum getrocknet und chromatographisch gereinigt (Kieselgel, CHCl₃/MeOH 19/1).
Ausbeute: 0.18 g= 15%
¹³C (100MHz, DMSO-d₆) δ 149.94, 147.70, 137.38, 137.26, 132.04, 130.41, 129.55, 129.19, 128.30, 127.71, 127.28, 126.23, 123.52, 122.87, 122.67, 114.71, 46.23, 40.92

### Beispiel 46: 3-(2,4-Dichlorphenoxy)-4-methylsulfonylaminobenzolsulfonsäure -(4-methoxyphenyl)amid

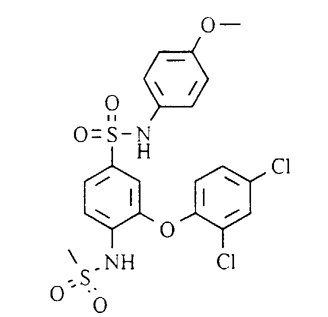

3-(2,4-Dichlorphenoxy)-4-methylsulfonylaminobenzolsulfonsäurechlorid (1.08g, 2.51 mmol) wurde in CH₂Cl₂ (5 ml) gelöst und bei 0°C zu einer Lösung von p-Anisidin (0 46 g, 3.7 mmol) in Pyridin (20 ml) zugetropft. Die Mischung wurde 1 h bei 0°C gerührt und dann abrotiert. Der Rückstand wurde im Vakuum getrocknet und chromatographisch gereinigt (Kieselgel, CH₂Cl₂/Ethylacetat 19/1).
Ausbeute: 0.37 g = 29%
¹³C (100MHz, DMSO-d₆) δ 156.96, 149.63, 147.53, 135.54, 132.26, 130.44, 129.82, 129.77, 129.13, 126.41, 124.11, 123.12, 123.07, 122.72, 114.57, 114.42, 55.31, 41.03

### Beispiel 47: 3-(2,4-Dichlorphenoxy)-4-methylsulfonylaminobenzolsulfonsäure-(2-pyridyl)amid

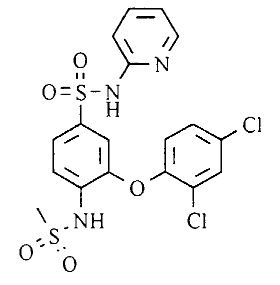

3-(2,4-Dichlorphenoxy)-4-methylsulfonylaminobenzolsulfonsäurechlorid (1.08 g, 2.51 mmol) wurde in CH₂Cl₂ (5 ml) gelöst und bei 0°C zu einer Lösung von 2-Aminopyridin (0.30 g, 3.2 mmol) in Pyridin (20 ml) zugetropft. Die Mischung wurde 1 h bei 0°C gerührt und dann abrotiert. Der Rückstand wurde im Vakuum getrocknet und chromatographisch gereinigt (Kieselgel, Chloroform/MeOH 19/1).
Ausbeute: 0.22 g = 18%
¹³C (100MHz, DMSO-d₆) (155.91, 150.50, 148.60, 146.84, 141.94, 139.86, 130.21, 129.08, 128.95, 127.90, 126.19, 125.43, 123.47, 121.39, 114.14, 112.17, 11.81, 40.66

### Beispiel 48: 3-(2,4-Dichlorphenoxy)-4-methylsulfonylaminobenzolsulfonsäure -(3-pyridyl)amid

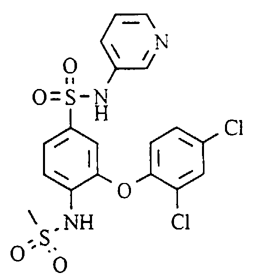

3-(2,4-Dichlorphenoxy)-4-methylsulfonylaminobenzolsulfonsäurechlorid (1.08 g, 2.51 mmol) wurde in CH₂Cl₂ (5 ml) gelöst und bei 0°C zu einer Lösung von3-Aminopyridin (0.30 g, 3.2 mmol) in Pyridin (20 ml) zugetropft. Die Mischung wurde 1 h bei 0°C gerührt und dann abrotiert. Der Rückstand wurde im Vakuum getrocknet und chromatographisch gereinigt (Kieselgel, Chloroform/MeOH 19/1).
Ausbeute: 0.18 g= 15%
¹³C (100MHz, DMSO-d₆) (149.43, 147.65, 145.75, 142.35, 134.92, 134.17, 132.57, 130.56, 130.03, 129.30, 128.21, 126.52, 124.12, 123.39, 122.90, 122.70, 114.05, 41.07

### Beispiel 49:

### [3-(2,4-Dichlorphenoxy)-4-methylsulfonylaminophenylsulfanoyl]-β-alanin

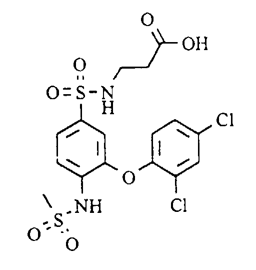

3-(2,4-Dichlorphenoxy)-4-methylsulfonylaminobenzolsulfonsäurechlorid (2.28 g, 5.3 mmol) wurde in Dioxan (5 ml) gelöst und bei 0°C zu einer Lösung von b-Alanin (3.77 g, 42.4 mmol) in 1N wässriger NaOH (42 ml, 42 mmol) und Dioxan (20 ml) zugetropft. Die Mischung wurde 3 h bei 0°C gerührt, mit conc. HCl angesäuert und mit Ethylacetat extrahiert. Die vereinigten organischen Phasen wurden über MgSO₄ getrocknet und das Lösungsmittel wurde abrotiert. Der Rückstand wurde chromatographisch gereinigt (Kieselgel, Petrolether/Ethylacetat/HOAc 1/1/0.1).
Ausbeute: 0.52 g = 20%
¹³C (100MHz, DMSO-d₆) δ 172.25, 150.01, 147.65, 136.43, 132.52, 130.41, 129.48, 129.18, 126.14, 123.38, 122.81, 122.78, 114.83, 40.97, 38.62, 34.20

### Beispiel 50: 3-(2,4-Dichlorphenoxy)-4-methylsulfonylaminobenzolsulfonsäure -2-hydroxyethylamid

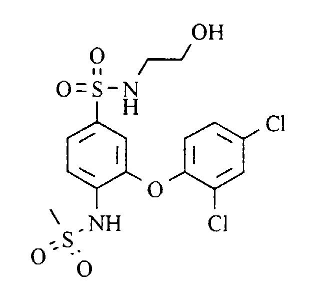

3-(2,4-Dichlorphenoxy)-4-methylsulfonylaminobenzolsulfonsäurechlorid (1.1g, 2.5 mmol) wurde in CH₂Cl₂ (5 ml) gelöst und bei 0°C zu einer Lösung von Ethanolamin (0.20 g, 3.3 mmol) in Pyridin (20 ml) zugetropft. Die Mischung wurde 1 h bei 0°C gerührt und dann abrotiert Der Rückstand wurde im Vakuum getrocknet und chromatographisch gereinigt (Kieselgel, Chloroform/MeOH 19/1).
Ausbeute: 0.28 g = 24%
¹³C (100MHz, DMSO-d₆) δ 149.97, 147.71, 137.12, 130.41, 129.53, 129.20, 126.19, 123.55, 123.52, 122.83, 122.66, 114.77, 59.95, 45.11, 41.01

### Beispiel 51:

### 3-(2,4-Dichlorphenoxy)-4-methylsulfonylaminobenzolsulfonsäure bis(hydroxymethyl)methylamid

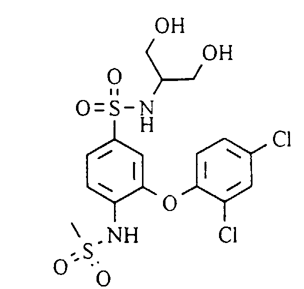

3-(2,4-Dichlorphenoxy)-4-methylsulfonylaminobenzolsulfonsäurechlorid (1.8g, 2.51 mmol) wurde in CH₂Cl₂ (5 ml) gelöst und bei 0°C zu einer Lösung von Bis(hydroxymethyl)methylamin (0.44 g, 4.8 mmol) in Pyridin (20 ml) zugetropft. Die Mischung wurde 2 h bei 0°C gerührt und dann abrotiert. Der Rückstand wurde im Vakuum getrocknet und chromatographisch gereinigt (Kieselgel, Chloroform/MeOH 19/1).
Ausbeute: 0.35 g = 29%
¹³C (100MHz, DMSO-d₆) δ 150.17, 147.50, 138.39, 132.09, 130.34, 129.29, 129.10, 126.02, 123.25, 122.75, 122.51, 115.22, 60.36, 57.07, 40.94

### Beispiel 52:

### 4[-3-(2,4-Dichlorphenoxy)-4-methylsulfonylaminophenylsulfamoyl-]-E-zimtsäure

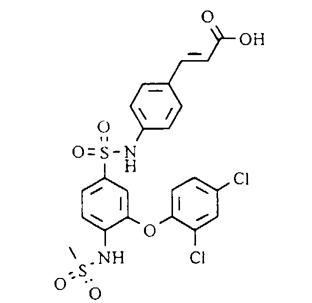

3-(2,4-Dichlorphenoxy)-4-methylsulfonylaminobenzolsulfonsäurechlorid (1.1g. 2.55 mmol) wurde in CH₂Cl₂ (5 ml) gelöst und bei 0°C zu einer Lösung von 4-Aminozimtsäure Hydrochlorid (0.65 g, 43.3 mmol) und DMAP (0.44 g, 3.6 mmol) in Pyridin (20 ml) zugetropft. Die Mischung wurde 7 h bei 0°C gerührt und dann abrotiert. Der Rückstand wurde im Vakuum getrocknet und chromatographisch gereinigt (Kieselgel Petrolether/Ethylacetat/HOAc 1/1/0.1).
Ausbeute: 0.43 g = 30%
¹³C (100MHz, DMSO-d₆) δ 167.68, 149.54, 147.56, 143.23, 139.25, 130.47, 130.33, 130.07, 129.89, 129.42, 129.21, 126.45, 123.19, 122.96, 122.71, 120,18, 118.42, 114.28, 113.74, 41.10

### Beispiel 53: 3-(2,4-Dichlorphenoxy)-4-methylsulfonylaminophenylsulfonsäure N-methylsulfonylamid

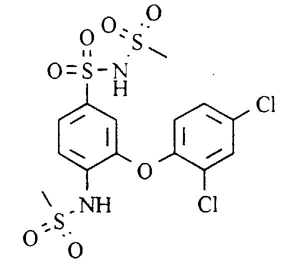

Methansulfonsäureamid (1.2 g, 12.5 mmol) wurde in abs. THF(50 ml) gelöst und TMEDA (7.55 ml) wurde zugesetzt. Die Lösung wurde auf -50°C gekühlt und BuLi (7.8 ml, 12.5 mmol) zugesetzt. Es wurde 15 min. bei dieser Temperatur gerührt dann eine Lösung von 3-(2,4-Dichlorphenoxy)-4-methylsulfonylaminobenzolsulfonsäurechlorid (0.5 g, 1.25 mmol) in THF (5 ml) zugetropft. Die Mischung wurde 4 h bei -40°C gerührt und durch Zugabe von Essigsäure gequencht. Die Mischung wurde in Ethylacetat gelöst und mit Wasser extrahiert . Die vereinigten organischen Phasen wurden über MgSO₄ getrocknet und das Lösungsmittel wurde abrotiert Der Rückstand wurde chromatographisch gereinigt (Kieselgel, CHCl₃/MeOH 18/3).
Ausbeute: 0.18 g = 29%
¹³C (100MHz, DMSO-d₆) δ 150.59, 147.38, 144.52, 130.19, 129.82, 128.93, 128.80, 125.72, 124.30, 122.50, 121.96, 115.67, 42.92, 40.84

### Beispiel 54: 3-(2,4-Dichlorphenoxy)-4-methylsulfonylaminophenylsulfonsäure N-formylamid

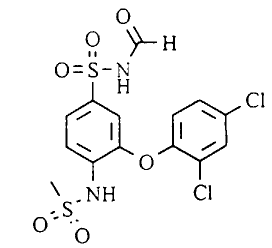

Formamid (0.5 ml, 12.5 mmol) wurde in abs. THF (50 ml) gelöst und TMEDA (7.55 ml) wurde zugesetzt. Die Lösung wurde auf -50°C gekühlt und BuLi (7.8 ml, 12.5 mmol) zugesetzt. Es wurde 15 min. bei dieser Temperatur gerührt dann eine Lösung von 3-(2,4-Dichlorphenoxy)-4-methylsulfonylaminobenzolsulfonsäurechlorid (0.5 g, 1.25 mmol) in THF (5 ml) zugetropft. Die Mischung wurde 5 h bei -40°C gerührt und durch Zugabe von Essigsäure gequencht. Die Mischung wurde in Ethylacetat gelöst und mit Wasser extrahiert . Die vereinigten organischen Phasen wurden über MgSO₄ getrocknet und das Lösungsmittel wurde abrotiert. Der Rückstand wurde chromatographisch gereinigt (Kieselgel, CHCl₃/MeOH 9/2).
Ausbeute: 0.04 g = 7%
¹³C (100MHz, MeOH-d₄) δ 166.05, 150.96, 149.39, 139.60, 133.78, 132.83, 132.16, 130.45, 128.84, 125.06, 123.82, 123.57, 115,15, 41.10

### Beispiel 55: 3-(2,4-Dichlorphenoxy)-4-methylsulfonylaminophenylsulfonsäure N-(N'-formyl)-hydrazid

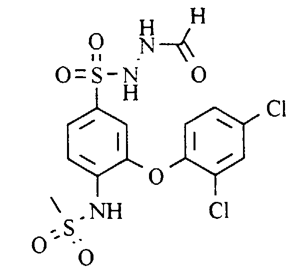

3-(2,4-Dichlorphenoxy)-4-methylsulfonylaminobenzolsulfonsäurechlorid (1.1 g, 2.5 mmol) wurde in CH₂Cl₂ (5 ml) gelöst und bei 0°C zu einer Lösung von Formylhydrazin (0.18 g, 3.0 mmol) in Pyridin (20 ml) zugetropft. Die Mischung wurde 1 h bei 0°C gerührt und dann abrotiert. Der Rückstand wurde im Vakuum getrocknet und chromatographisch gereinigt (Kieselgel, Chloroform/MeOH 19/1).
Ausbeute: 0.73 g = 64%
¹³C (100MHz, DMSO-d₆) δ 166.84, 159.45, 150.27, 150.15, 147.20, 146.95, 134.81, 133.25, 130.32, 129.27, 129.07, 125.82, 124.43, 124.19, 124.01, 123.03, 122.89, 122.33, 122,14, 116.68, 116.35, 40.95, 40.88

### Beispiel 56: 3-(3,4-Dichlorphenylthio)-4-methylsulfonylaminobenzolsulfonamid

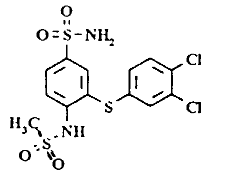

### Beispiel 57: 3-(3,4-Dichlorphenylthio)-4-methylsulfonylaminobenzolsulfonamid

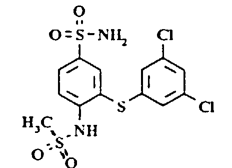

### Beispiel 58: 3-(2,4-Dimethylphenylthio)-4-methylsulfonylaminobenzolsulfonamid

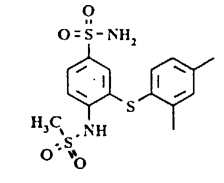

### Beispiel 59: 3-(2-Chlor-4-methylphenylthio)-4-methylsulfonylaminobenzolsulfonamid

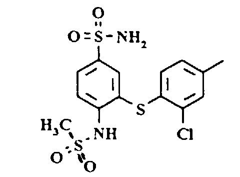

### Beispiel 60: 3-(2-Methyl-4-chlorphenylthio)-4-methylsulfonylaminobenzolsulfonamid

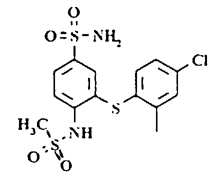

### Beispiel 61: 3-(2-Chlor-4-trifluormethylphenylthio)-4-methylsulfonylamino-benzolsulfonamid

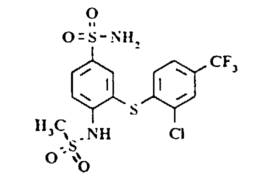

### Beispiel 62: 3-(2,6-Dichlorphenylthio)-4-methylsulfonylaminobenzolsulfonamid

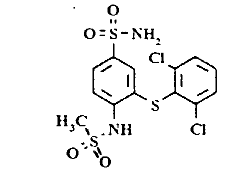

### Beispiel 63: 3-(2,4-Dichlorphenylamino)-4-methylsulfonylaminobenzolsulfonamid

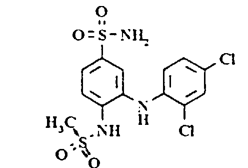

### Beispiel 64: 3-(4-Chlorphenylamino)-4-methylsulfonylaminobenzolsulfonamid

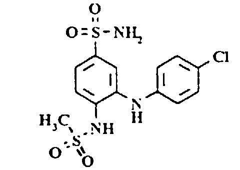

### Beispiel 65: 3-(2,4,6-Trichlorphenylamino)-4-methylsulfonylaminobenzolsulfonamid

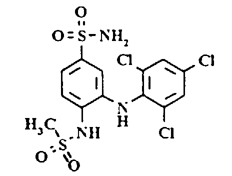

### Beispiel 66: 3-(2-Chlorphenylamino)-4-methylsulfonylaminobenzolsulfonamid

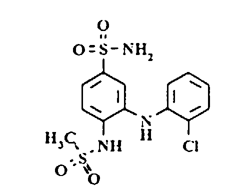

### Beispiel 67: 3-(3-Chlorphenylamino)-4-methylsulfonylaminobenzolsulfonamid

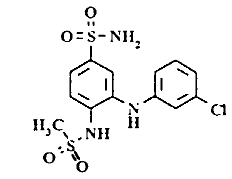

### Beispiel 68: 3-(2,6-Dichlorphenylamino)-4-methylsulfonylaminobenzolsulfonamid

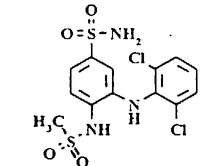

### Beispiel 69: 3-(2-Chlor-4-methoxyphenylamino)-4-methylsulfonylaminobenzolsulfonamid

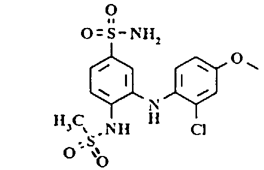

### Beispiel 70: 3-(2-Fluor-4-chlorphenylamino)-4-methylsulfonylaminobenzolsulfonamid

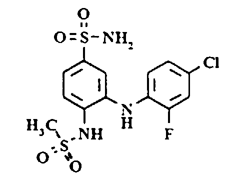

### Beispiel 71: 3-(4-Bromphenylamino)-4-methylsulfonylaminobenzolsulfonamid

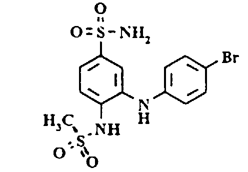

### Beispiel 72: 3-(4-Fluorphenylamino)-4-methylsulfonylaminobenzolsulfonamid

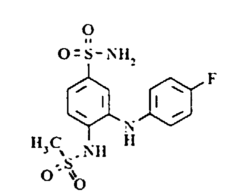

### Beispiel 73: 3-(3-Fluorphenylamino)-4-methylsulfonylaminobenzolsulfonamid

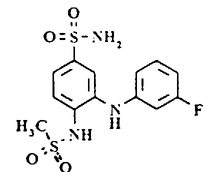

### Beispiel 74: 3-(2-Fluorphenylamino)-4-methylsulfonylaminobenzolsulfonamid

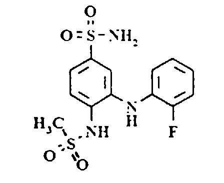

### Beispiel 75: 3-(2,4-Dichlorphenylthio)-4-trifluormethylsulfonylaminobenzolsulfonamid

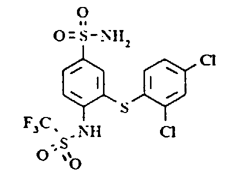

3-(2,4-Dichlorphenylthio)-4-amino-benzolsulfonamid (1.00g, 2.86mmol) wurde in CH₂Cl₂ (50ml) gelöst und Triethylamin (4.00ml, 28.6mmol) wurde zugegeben. Die Mischung wurde auf 0°C gekühlt und Trifluormethansulfonylchlorid (2.42ml, 22.9mmol) wurde zugetropft. Die Reaktionslösung wurde 30 min bei 0°C und anschließend 2 h bei Raumtemperatur gerührt. Es wurde mit IN HCl hydrolisiert, mit CH₂Cl₂ extrahiert und die vereinigten organischen Phasen über MgSO₄ getrocknet. Das Produkt wurde chromatographisch gereinigt (Kieselgel, CH₂Cl₂/MeOH 15/1).
Ausbeute: 0.15 g = 11%
¹³C (100MHz, CDCl₃)d 151.39, 136.07, 134.56, 133.88, 131.84, 131.62, 130.06, 129.51, 129.31, 127.58, 127.55, 126.20, 122.89, 119.58, 114.70, 111.42

### Beispiel 76: [3-(2,4-Dichlorphenylthio)-4-trifluormethylsulfonylaminobenzolsulfonamid] Natriumsalz

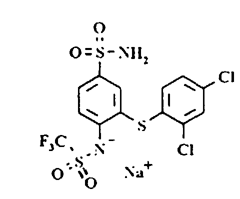

### Beispiel 77: 3-(2,4-Dichlorphenylthio)-4-trichlormethylsulfonylaminobenzolsulfonamid

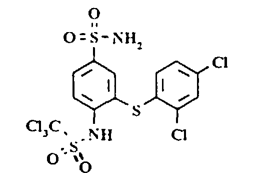

### Beispiel 78:: 3-(2,4-Dichlorphenylthio)-4-isopropylsulfonylaminobenzolsulfonamid

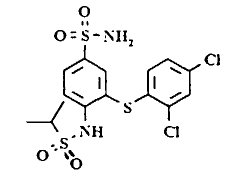

### Beispiel 79: 3-(2,4-Dichlorphenylthio)-4-ethylsulfonylaminobenzolsulfonamid

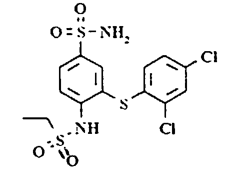

(a) 2-(2,4-Dichlorphenylthio)-N-ethylsulfonylanilid 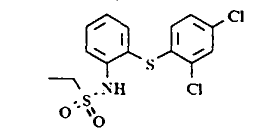 Die Synthese wurde analog zu Beispiel 1 (c ) durchgeführt. Das Produkt wurde chromatographisch gereinigt (Kieselgel, CH₂Cl₂).
   ¹³C (100MHz , CDCl₃)d 139.98, 137.64, 133.48, 132.73, 132.15, 129.77, 128.35, 127.88, 125.18, 118.99, 118.87, 46.73, 8.12
(b) 3-(2,4-Dichlorphenylthio)-4-ethylsulfonylaminobenzolsulfonamid 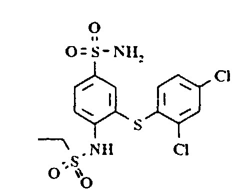 Die Synthese wurde analog zu Beispiel 1 (d) und (e) durchgeführt. Das Produkt wurde aus CH₂Cl₂ /McOH umkristallisiert.
   ¹³C (100MHz, DMSO-*d*₆)d 141.78, 141.11, 134.04, 132.89, 132.81, 132.03, 131.06, 129.71, 128.58, 127.23, 127.12, 125.20, 47.70, 8.11

### Beispiel 80: 3-(2,4-Dichlorphenylthio)-4-propylsulfonylaminobenzolsulfonamid

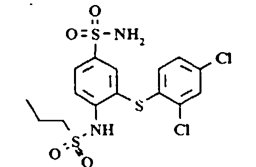

### Beispiel 81:: 3-(2,4-Dichlorphenylthio)-4-(2-chlorethylsulfonylamino)benzolsulfonamid

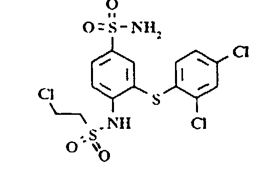

### Beispiel A

### Humaner COX-2 Test

Zellen einer humanen monocytoiden Zell-Linie werden mit LPS stimuliert (Brutschrank bei 37°C. 5% CO₂ angereicherte Atmosphäre und annähernd 100% Luftfeuchtigkeit), um COX-2 zu induzieren. Danach wird das Kulturmedium (RPMI 1640 angereichert mit 10% FCS, 2 mM Glutamin, 10 000 U/ml Penicillin, 10 ng/ml Streptomycin und 1 mM Pyruvat) erneuert und potentielle Hemmstoffe der Cyclooxygenase-2, gelöst in Kulturmedium oder in Phosphat gepufferte Saline oder in irgendeinem anderen Zellkultur-verträglichen Lösungsmittel, hinzugefügt und eine halbe Stunde wie oben beschrieben inkubiert. Arachidonsäure wird hinzupipettiert und 15 Minuten weiter inkubiert. Der Kulturüberstand der Zellen wird abgehoben und auf seinen Gehalt an Produkten des Cyclooxygenasestoffwechsels (z.B. Prostaglandin E2, Prostaglandin F₁ₐ, Thromboxan B₂) hin mittels ELISA gemessen.

### Beispiel B:

### Humaner COX 1- Test

Die Hemmung der Arachidonsäure-induzierten Aggregation von gewaschenen humanen Thrombozyten wurde als Testsystem für eine Abschätzung der Hemmung der Cyclooxygenase I verwendet. Die Testsubstanzen wurden einer Thrombozytensuspension bei 37°C zwei Minuten vor Zugabe der Arachidonsäure (10 µM Endkonzentration) zugesetzt und der Aggregationsverlauf mittels Aggregometer registriert. Mit Hilfe einer Konzentrations-Wirkungskurve wurde die Konzentration der Testsubstanz bestimmt, bei der 50% Aggregation gemessen wurden (IC50).

Die Ergebnisse der beiden Tests, sowie die daraus ermittelte Selektivität ist in Tabelle 1 angegeben.

**Tabelle 1**

| Verbindung | COX I IC50 µM | COX II IC50 µM | COX I/COX II |
|---|---|---|---|
| 1 | ≥50 | 0,10 | ≥500 |
| 4 | ≥45 | 0,11 | ≥450 |
| 6 | 52 | 0,2 | 260 |
| 7 | 27 | 0,027 | 1000 |
| 8 | 11 | 0,15 | 73 |
| 17 | 60 | 0,17 | 353 |
| 19 | ≥60 | 0,54 | ≥110 |
| 25 | ≥35 | 0,17 | ≥196 |
| 26 | ≥70 | 0,27 | ≥253 |

## Patentansprüche

1. Verbindungen der Formel I 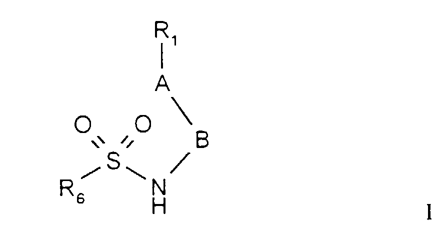 in der
A Sauerstoff Schwefel oder NH,
R₁ einen gegebenenfalls ein- oder mehrfach durch Halogen, Alkyl, CF₃ oder Alkoxy substituierter Cycloalkyl-, Aryl- oder Heteroarylrest
B eine Gruppe der Formel II a oder II b 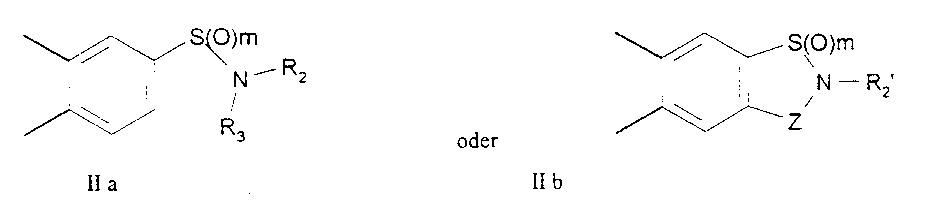
R₂ und R₃ unabhängig voneinander Wasserstoff, einen gegebenenfalls polyfluorierten Alkylrest. einen Aralkyl- Aryl- oder Heteroarylrest oder einen Rest (CH₂)ₙ-X,
oder
R₂ und R₃ gemeinsam mit dem N- Atom einen drei bis siebengliedrigen, gesättigten, teilweise oder vollständig ungesättigten Heterocyclus mit einem oder mehreren Heteroatomen N, O, oder S, der gegebenenfalls durch Oxo, einen Alkyl-, Alkylaryl- oder Arylrest oder einen Rest (CH₂)ₙ-X substituiert sein kann,
R₂' Wasserstoff, einen gegebenenfalls polyfluorierten Alkylrest, einen Aralkyl- Aryl- oder Heteroarylrest oder einen Rest (CH₂)ₙ-X,
wobei
X Halogen, NO_{2,} -OR₄, -COR₄, -CO₂R₄, - OCO₂R₄, -CN, -CONR₄OR₅, -CONR₄R₅,-SR₄, -S(O)R₄, -S(O)₂R₄, -NR₄R₅, -NHC(O)R₄, -NHS(O)₂R₄
Z -CH₂-, -CH₂-CH₂-, -CH₂-CH₂-CH₂-, -CH₂-CH=CH-, -CH=CH-CH₂-, -CH₂-CO-, -CO-CH₂₋, -NHCO-, - -CONH-, -NHCH₂₋, -CH₂NH-, -N=CH-, -NHCH-, -CH₂-CH₂-NH-, -CH=CH-, >N-R₃, >C=O, >S(O)ₘ,
R₄ und R₅ unabhängig voneinander Wasserstoff, Alkyl, Aralkyl oder Aryl,
n eine ganze Zahl von 0 bis 6,
R₆ einen geradkettigen oder verzweigten Alkylrest mit 1 - 4 C- Atomen, der gegebenenfalls durch Halogen oder Alkoxy ein- oder mehrfach substituiert sein kann, oder CF₃ und
m eine ganze Zahl von 0 bis 2
bedeuten,
mit der Bedingung, daß A nicht O bedeutet, wenn R₆ CF₃ bedeutet,
und deren pharmazeutisch verträgliche Salze.

2. Verbindungen der Formel I nach Anspruch 1, in der R₁ einen gegebenenfalls ein- oder mehrfach durch Halogen, Methoxy, Methyl oder Ethyl substituierten Cyclopentyl-, Cyclohexyl-, Phenyl-, Pyridyl-, Thienyl- oder Thiazolylrest bedeutet.

3. Verbindungen der Formel I nach Anspruch 2, in der R₁ einen Cyclohexylrest, einen Phenylrest. einen 2,4-Dichlorphenylrest oder einen 2,4-Difluorphenylrest bedeutet

4. Pharmazeutische Zusammensetzung enthaltend als Wirkstoff mindestens eine Verbindung der allgemeinen Formel I nach Anspruch 1.

5. Verwendung von Verbindungen der Formel I 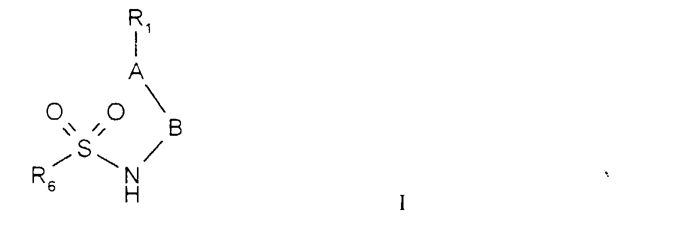 in der
A Sauerstoff Schwefel oder NH,
R₁ einen gegebenenfalls ein- oder mehrfach durch Halogen, Alkyl, CF₃ oder Alkoxy substituierter Cycloalkyl-, Aryl- oder Heteroarylrest
B eine Gruppe der Formel II a oder II b 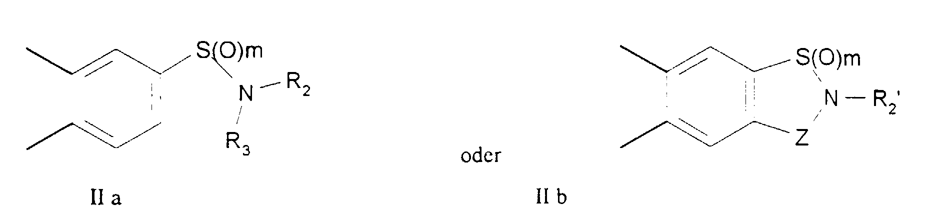
R₂ und R₃ unabhängig voneinander Wasserstoff, einen gegebenenfalls polyfluorierten Alkylrest, einen Aralkyl- Aryl- oder Heteroarylrest oder einen Rest (CH₂)ₙ-X,
oder
R₂ und R₃ gemeinsam mit dem N- Atom einen drei bis siebengliedrigen, gesättigten, teilweise oder vollständig ungesättigten Heterocyclus mit einem oder mehreren Heteroatomen N, O, oder S, der gegebenenfalls durch Oxo, einen Alkyl-, Alkylaryl- oder Arylrest oder einen Rest (CH₂)ₙ-X substituiert sein kann,
R₂' Wasserstoff, einen gegebenenfalls polyfluorierten Alkylrest, einen Aralkyl- Aryl- oder Heteroarylrest oder einen Rest (CH₂)ₙ-R
wobei
X Halogen, NO₂, -OR₄, -COR₄, -CO₂R₄, -OCO₂R₄, -CN, -CONR₄OR₅, -CONR₄R₅,-SR₄, -S(O)R₄, -S(O)₂R₄, -NR₄R₅, -NHC(O)R₄, -NHS(O)₂R₄
Z -CH₂-, -CH₂-CH₂-, -CH₂-CH₂-CH₂-, -CH₂-CH=CH-, -CH=CH-CH₂-, -CH₂-CO-, -CO-CH₂-, -NHCO-, - -CONH-, -NHCH₂₋, -CH₂NH-, -N=CH-, -NHCH-, -CH₂-CH₂-NH-, -CH=CH-, >N-R₃, >C=O, >S(O)ₘ,
R₄ und R₅ unabhängig voneinander Wasserstoff, Alkyl, Aralkyl oder Aryl,
n eine ganze Zahl von 0 bis 6,
R₆ einen geradkettigen oder verzweigten Alkylrest mit 1 - 4 C- Atomen, der gegebenenfalls durch Halogen oder Alkoxy ein- oder mehrfach substituiert sein kann, und
m eine ganze Zahl von 0 bis 2
bedeuten,
und deren pharmazeutisch verträgliche Salze zur Herstellung eines Mittel zu Behandlung und Linderung von Krankheiten oder Störungen, die durch Hemmung des Enzym Cyclooxygenase II geheilt oder gelindert werden können.

6. Verwendung von Verbindungen der Formel I nach Anspruch 5 zur Herstellung eines Mittels zur Behandlung oder Linderung von entzündlichen Prozessen.

7. Verwendung von Verbindungen der Formel I nach Anspruch 5 zur Herstellung eines als Mittels zur Behandlung von Schmerzen.

## Claims

1. Compounds of formula I 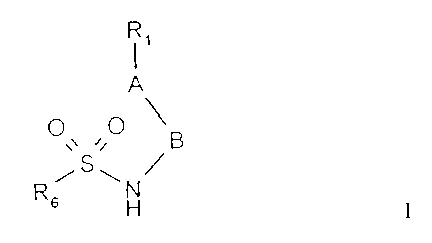 wherein
A denotes oxygen, sulphur or NH,
R₁ denotes a cycloalkyl, aryl or heteroaryl group optionally mono- or polysubstituted by halogen, alkyl, CF₃ or alkoxy
B denotes a group of formula IIa or IIb 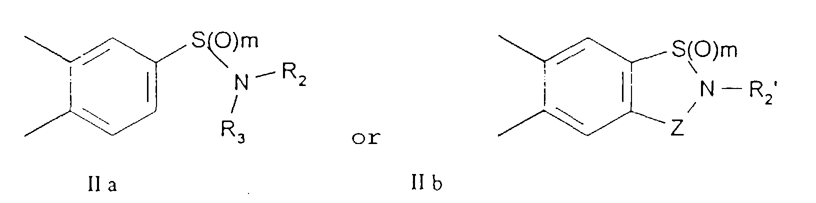
R₂ and R₃ independently of each other denote hydrogen, an optionally polyfluorinated alkyl radical, an aralkyl, aryl or heteroaryl radical or a radical (CH₂)ₙ-X,
or
R₂ and R₃ together with the N-atom denote a three- to seven-membered, saturated, partially or totally unsaturated heterocycle with one or more heteroatoms N, O or S, which may optionally be substituted by oxo, an alkyl, alkylaryl or aryl group or a group (CH₂)ₙ-X, R₂' denotes hydrogen, an optionally polyfluorinated alkyl group, an aralkyl, aryl or heteroaryl group or a group (CH₂)ₙ-X,
wherein
X denotes halogen, NO₂, -OR₄, -COR₄, -CO₂R₄, -OCO₂R₄, -CN, -CONR₄OR₅, -CONR₄R₅, -SR₄, -S(O)R₄, -S(O)₂R₄, -NR₄R₅, -NHC(O)R₄, -NHS(O)₂R₄
Z denotes -CH₂-, -CH₂-CH₂-, -CH₂-CH₂-CH₂-, -CH₂-CH=CH-, -CH=CH-CH₂-, -CH₂-CO-, -CO-CH₂-, -NHCO-, -CONH-, -NHCH₂-, -CH₂NH-, -N=CH-, -NHCH-, -CH₂-CH₂-NH-, -CH=CH-, >N-R₃, >C=O, >S(Q)ₘ,
R₄ and R₅ independently of each other denote hydrogen, alkyl, aralkyl or aryl,
n is an integer from 0 to 6,
R₆ is a straight-chained or branched C₁₋₄-alkyl group which may optionally be mono- or polysubstituted by halogen or alkoxy, or R₆ denotes CF₃, and
m denotes an integer from 0 to 2,
with the proviso that A does not represent O if R₆ denotes CF₃,
and the pharmaceutically acceptable salts thereof.

2. Compounds of formula I according to claim 1, wherein R₁ denotes a cyclopentyl, cyclohexyl, phenyl, pyridyl, thienyl or thiazolyl group optionally mono- or polysubstituted by halogen, methoxy, methyl or ethyl.

3. Compounds of formula I according to claim 2, wherein R₁ denotes a cyclohexyl group, a phenyl group, a 2,4-dichlorophenyl group or a 2,4-difluorophenyl group.

4. Pharmaceutical composition containing as active substance at least one compound of general formula I according to claim 1.

5. Use of compounds of formula I 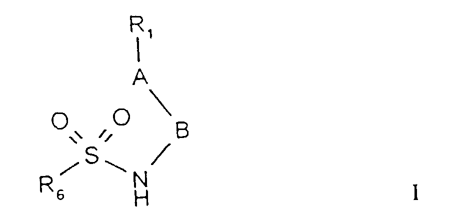 wherein
A denotes oxygen, sulphur or NH,
R₁ denotes a cycloalkyl, aryl or heteroaryl group optionally mono- or polysubstituted by halogen, alkyl, CF₃ or alkoxy
B denotes a group of formula IIa or IIb 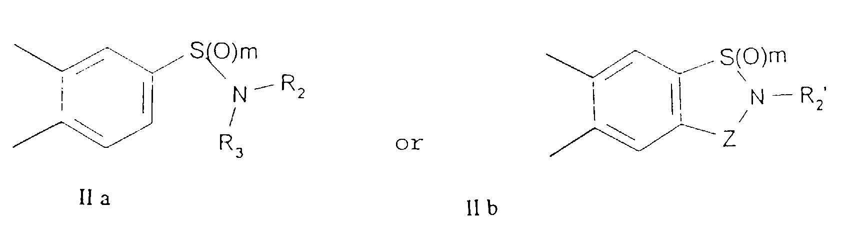
R₂ and R₃ independently of each other denote hydrogen, an optionally polyfluorinated alkyl radical, an aralkyl, aryl or heteroaryl radical or a radical (CH₂)ₙ-X,
or
R₂ and R₃ together with the N-atom denote a three- to seven-membered, saturated, partially or totally unsaturated heterocycle with one or more heteroatoms N, O or S, which may optionally be substituted by oxo, an alkyl, alkylaryl or aryl group or a group (CH₂)ₙ-X, R₂' denotes hydrogen, an optionally polyfluorinated alkyl group, an aralkyl, aryl or heteroaryl group or a group (CH₂)ₙ-X,
wherein
X denotes halogen, NO₂, -OR₄, -COR₄, -CO₂R₄, -OCO₂R₄, -CN, -CONR₄OR₅, -CONR₄R₅, -SR₄, -S(O)R₄, -S(O)₂R₄, -NR₄R₅, -NHC(O)R₄, -NHS(O)₂R₄
Z denotes -CH₂-, -CH₂-CH₂-, -CH₂-CH₂-CH₂-, -CH₂-CH=CH-, -CH=CH-CH₂-, -CH₂-CO-, -CO-CH₂-, -NHCO-, -CONH-, -NHCH₂-, -CH₂NH-, -N=CH-, -NHCH-, -CH₂-CH₂-NH-, -CH=CH-, >N-R₃, >C=O, >S(O)ₘ,
R₄ and R₅ independently of each other denote hydrogen, alkyl, aralkyl or aryl,
n is an integer from 0 to 6,
R₆ is a straight-chained or branched C₁₋₄-alkyl group which may optionally be mono- or polysubstituted by halogen or alkoxy, and
m denotes an integer from 0 to 2,
and the pharmaceutically acceptable salts thereof for the manufacture of a medicament for the treatment and alleviation of diseases or disorders which can be cured or alleviated by inhibiting the enzyme cyclooxygenase II.

6. Use of compounds of formula I according to claim 5 for the manufacture of a medicament for treating or alleviating inflammatory processes.

7. Use of compounds of formula I according to claim 5 for the manufacture of a medicament for treating pain.

## Revendications

1. Composés de formule 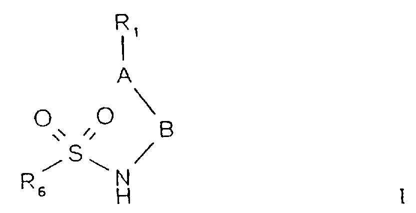 dans laquelle
A représente un atome d'oxygène, de soufre ou NH,
R₁ représente un radical cycloalkyle, aryle ou hétéroaryle éventuellement une ou plusieurs fois substitué par un ou des atomes d'halogène ou groupes alkyle, CF₃ ou alcoxy,
B représente un groupe de formule IIa ou IIb, 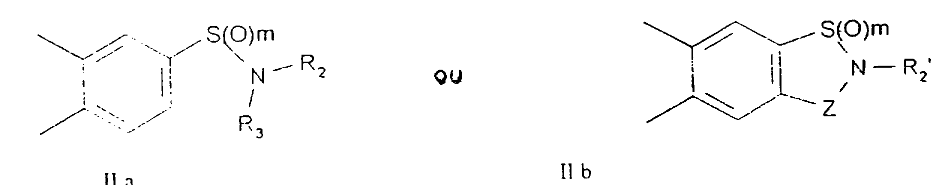
R₂ et R₃ représentent, indépendamment l'un de l'autre, un atome d'hydrogène, un radical alkyle éventuellement polyfluoré, un radical aralkyle, aryle ou hétéroaryle ou un radical (CH₂)ₙ-X;
ou
R₂ et R₃ forment ensemble, avec l'atome d'azote, un hétérocycle à 3-7 chaînons, saturé, partiellement ou totalement insaturé, comportant un ou plusieurs hétéroatomes N, O ou S, qui peut éventuellement être substitué par un groupe oxo, alkyle, alkylaryle, aryle ou par un groupe (CH₂)ₙ-X,
R₂' représente un atome d'hydrogène ou un radical alkyle éventuellement polyfluoré, un radical aralkyle, aryle ou hétéroaryle, ou un radical (CH₂)ₙ-X,
X représentant un atome d'halogène ou un groupe NO₂, -OR₄, -COR₄, -CO₂R₄, -OCO₂R₄, -CN, -CONR₄OR₅, -CONR₄R₅, -SR₄, -S(O)R₄, -S(O)₂R₄, -NR₄R₅, -NHC(O)R₄, -NHS(O)₂R₄,
Z représente -CH₂-, -CH₂-CH₂-, -CH₂-CH₂-CH₂-, -CH₂ -CH=CH-, -CH=CH-CH₂-, -CH₂-CO-, -CO-CH₂-, -NHCO-, -CONH-, -NHCH₂-, -CH₂NH-, -N=CH-, -NHCH-, -CH₂-CH₂-NH-, -CH=CH-, >N-R₃, >C=O, >S(O)ₘ,
R₄ et R₅ représentent, indépendamment l'un de l'autre, un atome d'hydrogène ou un groupe alkyle, aralkyle ou aryle,
n représente un nombre entier allant de 0 à 6,
R₆ représente un radical alkyle à chaîne droite ou ramifié, ayant de 1 à 4 atomes de carbone, qui peut éventuellement être une ou plusieurs fois substitué par un ou des atomes d'halogène ou groupes alcoxy, ou représente CF₃ et
m est un nombre entier allant de 0 à 2,
étant entendu que A ne représente pas un atome d'oxygène lorsque R₆ représente CF₃,
et leurs sels pharmaceutiquement acceptables.

2. Composés de formule I selon la revendication 1, dans lesquels R₁ représente une radical cyclopentyle, cyclohexyle, phényle, pyridyle, thiényle ou thiazolyle éventuellement une ou plusieurs fois substitué par un ou des atomes d'halogène ou groupes méthoxy, méthyle ou éthyle.

3. Composés de formule I selon la revendication 2, dans lesquels R₁ représente le radical cyclohexyle, phényle, 2,4-dichlorophényle ou 2,4-difluorophényle.

4. Composition pharmaceutique, contenant en tant que substance active au moins un composé de formule générale selon la revendication 1.

5. Utilisation de composés de formule I 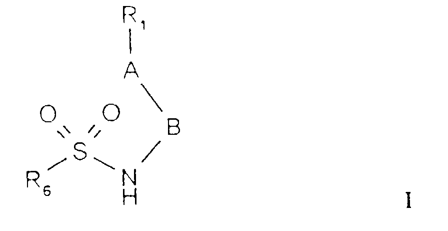 dans laquelle
A représente un atome d'oxygène, de soufre ou NH,
R₁ représente un radical cycloalkyle, aryle ou hétéroaryle éventuellement une ou plusieurs fois substitué par un ou des atomes d'halogène ou groupes alkyle, CF₃ ou alcoxy,
B représente un groupe de formule IIa ou IIb, 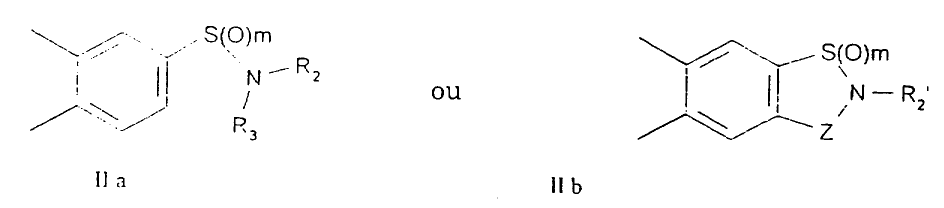
R₂ et R₃ représentent, indépendamment l'un de l'autre, un atome d'hydrogène, un radical alkyle éventuellement polyfluoré, un radical aralkyle, aryle ou hétéroaryle ou un radical (CH₂)ₙ-X,
ou
R₂ et R₃ forment ensemble, avec l'atome d'azote, un hétérocycle à 3-7 chaînons, saturé, partiellement ou totalement insaturé, comportant un ou plusieurs hétéroatomes N, O ou S, qui peut éventuellement être substitué par un groupe oxo, alkyle, alkylaryle, aryle ou par un groupe (CH₂)ₙ-X,
R₂' représente un atome d'hydrogène ou un radical alkyle éventuellement polyfluoré, un radical aralkyle, aryle ou hétéroaryle, ou un radical (CH₂)ₙ-X,
X représentant un atome d'halogène ou un groupe NO₂, -OR₄, -COR₄, -CO₂R₄, -OCO₂R₄, -CN, -CONR₄OR₅, -CONR₄R₅, -SR₄, -S(O)R₄, -S(O)₂R₄, -NR₄R₅, -NHC(O)R₄, -NHS(O)₂R₄,
Z représente -CH₂-, -CH₂-CH₂-, -CH₂-CH₂-CH₂-, -CH₂-CH=CH-, -CH=CH-CH₂-, -CH₂-CO-, -CO-CH₂-, -NHCO-, -CONH-, -NHCH₂-, -CH₂NH-, -N=CH-, -NHCH-, -CH₂-CH₂-NH-, -CH=CH-, >N-R₃, >C=O, >S(O)ₘ,
R₄ et R₅ représentent, indépendamment l'un de l'autre, un atome d'hydrogène ou un groupe alkyle, aralkyle ou aryle,
n représente un nombre entier allant de 0 à 6,
R₆ représente un radical alkyle à chaîne droite ou ramifié, ayant de 1 à 4 atomes de carbone, qui peut éventuellement être une ou plusieurs fois substitué par un ou des atomes d'halogène ou groupes alcoxy, et
m est un nombre entier allant de 0 à 2,
et de leurs sels pharmaceutiquement acceptables, pour la fabrication d'un médicament destiné au traitement ou à l'atténuation de maladies ou de troubles qui peuvent être atténués ou guéris par inhibition de l'enzyme cyclo-oxygénase II.

6. Utilisation des composés de formule I selon la revendication 5, pour la fabrication d'un médicament destiné au traitement ou à l'atténuation de processus inflammatoires.

7. Utilisation des composés de formule I selon la revendication 5, pour la fabrication d'un médicament destiné au traitement de douleurs.
